# EUROPÄISCHE PATENTSCHRIFT

(11) **EP 2 302 437 B1**
(45) Veröffentlichungstag und Bekanntmachung des Hinweises auf die Patenterteilung: **09.07.2014**
(21) Anmeldenummer: 10014151.4
(22) Anmeldetag: 12.01.2007
(51) Int. Cl.: G02B 21/22

(54) **Stereoskopisches optisches System**
Stereoscopic optical system
Système optique stéréoscopique

(30) Priorität: 13.01.2006 DE 102006001888
(43) Veröffentlichungstag der Anmeldung: 30.03.2011
(62) Teilanmeldung aus: 07702733.2
(73) Patentinhaber: Carl Zeiss Meditec AG, 07745 Jena (DE)
(72) Erfinder: Obrebski, Andreas, 40489 Düsseldorf (DE)
(74) Vertreter: Diehl & Partner GbR

(56) Entgegenhaltungen:
- CH-A5- 560 908
- DE-A1- 10 134 896
- DE-A1- 10 316 242
- JP-A- 2005 315 936

## Beschreibung

Die vorliegende Erfindung betrifft ein stereoskopisches optisches System.

Ein stereoskopisches optisches System weist üblicherweise wenigstens ein erstes optisches Teilsystem mit einer Mehrzahl von optischen Elementen zur Bereitstellung eines ersten Strahlengangs und ein zweites optisches Teilsystem mit einer Mehrzahl von optischen Elementen zur Bereitstellung eines zweiten Strahlengangs auf. Somit werden der erste und der zweite Strahlengang von wenigstens den ersten und zweiten optischen Teilsystemen getrennt geführt.

Da die beiden Strahlengänge üblicherweise dem linken bzw. rechten Auge eines Benutzers zugeordnet sind, werden die beiden ersten und zweiten Strahlengänge häufig auch als linker bzw. rechter Strahlengang bezeichnet. Statt dem Auge eines Benutzers können die beiden Strahlengänge jedoch beispielsweise auch einem ersten bzw. einem zweiten photosensitiven ortsauflösenden Halbleiterelement zugeführt werden. Bei den ersten und zweiten Halbleiterelementen kann es sich beispielsweise um CCD-Chips handeln. Derartige stereoskopische optische Systeme werden insbesondere als Stereokamera oder digitales Operationsmikroskop verwendet.

Kennzeichnend für derartige stereoskopische optische Systeme ist, daß die von den beiden Strahlengängen geführten Strahlenbündel miteinander in einem Fokussierpunkt außerhalb des stereoskopischen optischen Systems einen Stereowinkel α einschließen. Dabei muss es das stereoskopische optische System ermöglichen, daß sich die von den beiden Strahlengängen geführten Strahlenbündel auch nach einer Änderung eines Arbeitsabstands unter Einschluß eines neuen, von Null verschiedenen Winkels in einem neuen Fokussierpunkt schneiden.

Aus dem Dokument JP 2005315936 ist ein Feldstecher bekannt, bei dem die Vergrößerung dadurch zweistufig verändert werden kann, dass eine Linse in den linken bzw. rechten Abbildungsstrahlengang hinein bzw. hinaus bewegt werden kann. Die Verlagerung der Linse erfolgt dabei orthogonal zu dem jeweiligen Abbildungsstrahlengang.

Aus dem Dokument DE 101 34 896 ist eine Kopflupe mit den Merkmalen des Oberbegriffs des unabhängigen Anspruchs 1 bekannt, welche durch zwei verlagerbare Objektivlinsen eine Anpassung des Arbeitsabstandes erlaubt. Weiter ist ein Zoomsystem vorgesehen, um eine Abbildungsvergrößerung zu ändern.

Das Dokument CH 560 908 offenbart einen Binokulartubus mit einer Eingangsschnittweite von ∝.

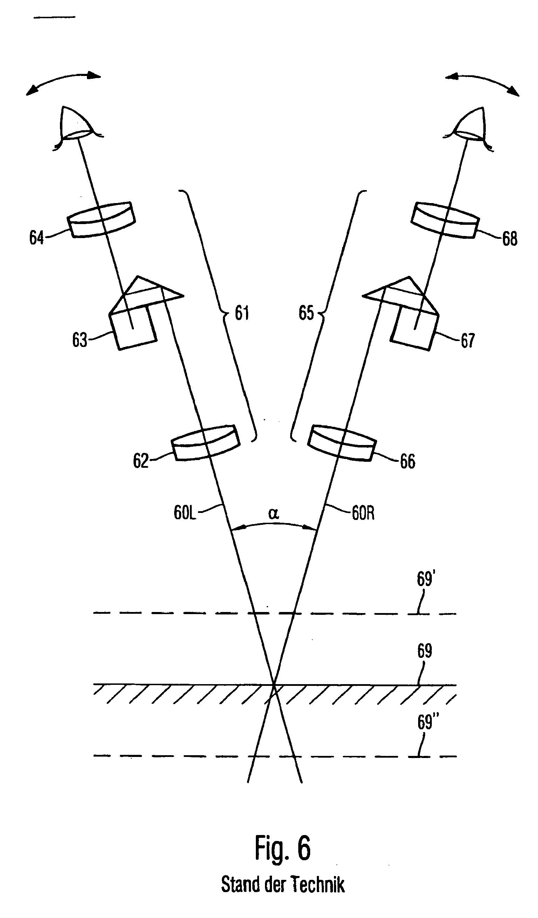

Ein als Greenough-System bekanntes optisches System ist schematisch in Figur 6 gezeigt.

Das gezeigte Greenough-System weist ein erstes optisches Teilsystem 61 mit einer Mehrzahl von optischen Elementen 62, 63, 64 zur Bereitstellung eines ersten Strahlengangs 60L und ein zweites optisches Teilsystem 65 mit einer Mehrzahl von optischen Elementen 66, 67, 68 zur Bereitstellung eines zweiten Strahlengangs 60R auf. Somit werden der erste und der zweite Strahlengang 60L, 60R von den ersten und zweiten optischen Teilsystemen 61, 65 getrennt geführt. Die beiden optischen Teilsysteme 61, 65 und damit auch die beiden Strahlengänge 60L, 60R sind relativ zueinander um einen einstellbaren Winkel α geneigt. In der Folge schneiden sich auch von den beiden Strahlengängen 60L, 60R jeweils geführte erste und zweite Strahlenbündel in einer Objektebene 69 unter einem einstellbaren Stereowinkel α.

Problematisch bei dem in Figur 6 gezeigten Greenough-System ist, daß der von den beiden Strahlengängen 60L, 60R eingeschlossene Winkel α nach einer Änderung eines Arbeitsabstands manuell oder mechanisch angepaßt werden muß. Eine derartige Änderung des Arbeitsabstandes und damit eine Verlagerung der Objektebene 69' bzw. 69" ist in Figur 6 gestrichelt dargestellt. Ersichtlich muß die relative Neigung der optischen Teilsysteme 61,65 angepasst werden, damit sich die von den Strahlengängen 60L, 60R der Teilsysteme 61, 65 geführten Strahlenbündel unter einem neuen Stereowinkel in der neuen Objektebene 69', 69" schneiden.

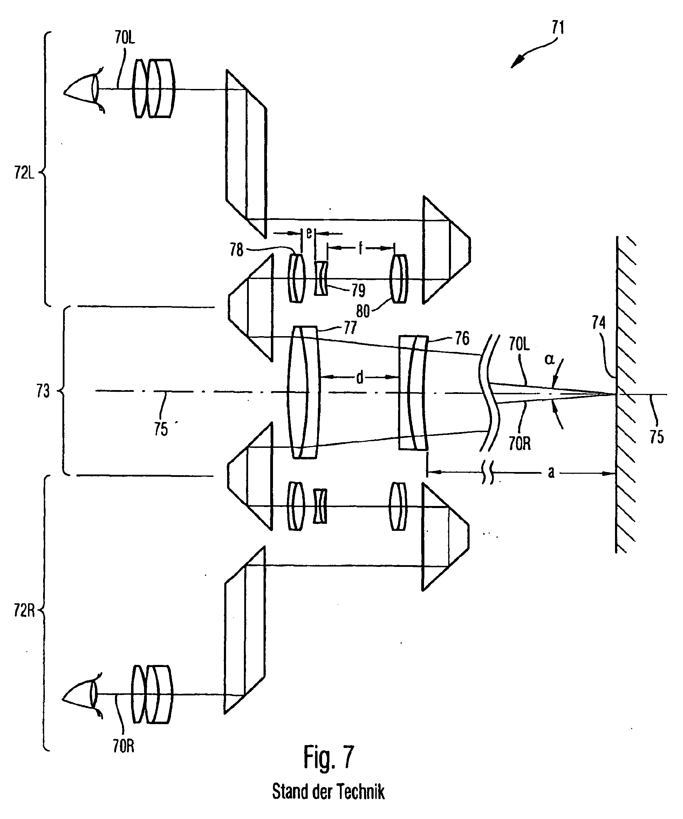

Zur Lösung dieses Problems ist aus der deutschen Patentanmeldung DE 101 34 896 A1 ein stereoskopisches optisches System bekannt, in dem die von Strahlengängen eines ersten und zweiten optischen Teilsystems getrennt geführten Strahlenbündel in einem Strahlengang eines dritten optischen Teilsystems gemeinsam geführt werden. Ein Strahlengang durch dieses vorbekannte optische System ist in Figur 7 dargestellt.

Das stereoskopische optische System 71 nach dem Stand der Technik weist zwei erste und zweite optische Teilsysteme (Okularsysteme) 72L, 72R auf, in deren jeweiligen Strahlengängen erste und zweite Teilstrahlenbündel 70L, 70R getrennt geführt werden. Weiter weist das stereoskopische optische System ein drittes optisches Teilsystem (Objektivsystem) 73 auf, in dessen Strahlengang die von den ersten und zweiten optischen Teilsystemen 72L, 72R getrennt geführten Teilstrahlenbündel 70L, 70R gemeinsam geführt werden. Die von den ersten und zweiten optischen Teilsystemen 72L, 72R getrennt geführten Teilstrahlenbündel 70L, 70R werden durch das dritte optische Teilsystem 73 derart abgebildet, daß sie sich in einer Objektebene 74 treffen und dabei miteinander einen Stereowinkel α einschließen.

Das optische System 71 ist für die Teilstrahlenbündel 70L und 70R bezüglich einer gemeinsamen Symmetrieachse 75 des optischen Systems 71 symmetrisch aufgebaut. Die von der Objektebene 74 kommenden Teilstrahlenbündel 70L, 70R treten durch eine gemeinsame optische Haupteintrittslinse 76 in das dritte optische Teilsystem 73 des stereoskopischen optischen Systems 71 ein.

Dabei werden die beiden Teilstrahlenbündel 70L, 70R in dem dritten optischen Teilsystem 73 gemeinsam so geführt, daß sie sich zumindest nicht vollständig überlappen, sondern das dritte optische Teilsystem 73 in unterschiedlichen Bereichen der verwendeten optischen Linsen 76, 77 durchdringen.

Nach ihrem Austreten aus dem dritten optischen Teilsystem 73 werden die Teilstrahlenbündel 70L, 70R gefaltet und treten jeweils in eines von dem ersten oder zweiten optischen Teilsystem 72L, 72R ein, wobei jedem der beiden Teilstrahlenbündel 70L, 70R vorzugsweise ein eigener Strahlengang eines eigenen ersten oder zweiten optischen Teilsystems 72L, 72R zugeordnet ist.

Durch Änderung von Linsenabständen e und f bzw. d zwischen optischen Linsen 76 bis 80 des stereoskopischen optischen Systems 71 ist eine variable Vergrößerung (Zoomfunktion) eines in der Objektebene 74 angeordneten betrachteten Objekts beziehungsweise eine Änderung des Arbeitsabstandes a (Fokussierung) zur Anpassung an ein in der Objektebene 74 angeordnetes betrachtetes Objekt möglich. Dabei stellt die gemeinsame Verwendung des dritten optischen Teilsystems 73 für die beiden Teilstrahlenbündel 70L und 70R durch Anpassung des Abstandes d sicher, daß sich Hauptstrahlen der Teilstrahlenbündel 70L, 70R auch nach einer Abstandsänderung (Fokussierung) immer in der Objektebene 74 treffen und dabei einen von Null verschiedenen Stereowinkel α einschließen. Dies erreicht der Fachmann durch geeignete Wahl der optischen Oberflächen der optischen Linsen 76, 77 des dritten optischen Teilsystems 73.

Der Inhalt der deutschen Patentanmeldung DE 101 34 896 A1, auf den vollumfänglich Bezug genommen wird, soll Teil der Offenbarung der vorliegenden Patentanmeldung sein.

Bei dem vorstehend beschriebenen stereoskopischen System ist es nachteilig, daß das gemeinsame dritte optische Teilsystem ein sehr großes Gewicht aufweist. Der Grund ist, daß die beiden Teilstrahlenbündel in dem dritten optischen Teilsystem so geführt werden müssen, daß sie die optischen Linsen des dritten optischen Teilsystems zumindest teilweise in unterschiedlichen Bereichen durchdringen. Weiter müssen die optischen Linsen des dritten optischen Teilsystems eine gewisse Verlagerung, Vergrößerung und/oder Verkleinerung eines jeweiligen Durchdringungsbereiches der beiden Teilstrahlenbündel erlauben. In der Folge müssen die gemeinsam für die beiden Teilstrahlenbündel verwendeten optischen Linsen des dritten optischen Teilsystems verglichen mit optischen Linsen der ersten und zweiten optischen Teilsysteme sehr groß ausgebildet sein.

Weiter führt die Anordnung der optischen Linsen des dritten optischen Teilsystems entlang der gemeinsamen Symmetrieachse des optischen Systems zu einer großen Baulänge des dritten optischen Teilsystems.

Bei Verwendung eines derartigen stereoskopischen Systems als Kopflupe hat das große Gewicht und die große Baulänge des dritten optischen Teilsystems eine erhebliche Beeinträchtigung der Bewegungsfreiheit eines Benutzers zur Folge. Bei einer Änderung von Linsenabstände verlagert sich zudem ein Schwerpunkt des optischen Systems und damit ein am Kopf des Benutzers angreifender Hebelarm. Außerdem führt das hohe Gewicht zusammen mit der großen Baulänge oft zu einer vorzeitigen Ermüdung des Benutzers und einer Verkrampfung der Nackenmuskulatur.

Es ist daher eine Aufgabe der vorliegenden Erfindung, ein stereoskopisches optisches System zur Verfügung zu stellen, welches ein niedriges Gewicht und eine große Kompaktheit aufweist und gleichzeitig automatisch sicherstellen kann, daß in einem Fokussierpunkt innerhalb eines Arbeitsbereichs des Systems immer ein Stereowinkel eingeschlossen wird. Weiter ist es Aufgabe der vorliegenden Erfindung, eine Kopflupe mit einem verbesserten Tragekomfort zur Verfügung zu stellen.

Die vorstehende Aufgabe wird durch die Kombination der Merkmale des unabhängigen Anspruchs 1 gelöst.

Bevorzugte Weiterbildungen finden sich in den Unteransprüchen.

Gemäß einer Ausführungsform weist ein stereoskopisches optisches System ein erstes optisches Teilsystem mit einer Mehrzahl von optischen Elementen zur Bereitstellung eines linken Abbildungsstrahlengangs des stereoskopischen optischen Systems und ein zweites optisches Teilsystem mit einer Mehrzahl von optischen Elementen zur Bereitstellung eines rechten Abbildungsstrahlengangs des stereoskopischen optischen Systems auf. Dabei weist das erste optische Teilsystem wenigstens eine erste optische Linse mit einer ersten optischen Oberfläche auf, die im Bereich des linken Abbildungsstrahlengangs eine Teilfläche einer ersten rotationssymmetrischen mathematischen Fläche ist. Die erste mathematische Fläche weist bezogen auf eine erste Symmetrieachse einen ersten maximalen Radius auf. Auch das zweite optische Teilsystem weist wenigstens eine zweite optische Linse mit einer zweiten optischen Oberfläche auf, die im Bereich des rechten Abbildungsstrahlengangs eine Teilfläche einer zweiten rotationssymmetrischen mathematischen Fläche ist. Die zweite mathematische Fläche weist bezogen auf eine zweite Symmetrieachse einen zweiten maximalen Radius auf. Die erste optische Linse ist eine erste off-axis Linse, deren erster Flächenschwerpunkt ihrer ersten Oberfläche von der ersten Symmetrieachse der ersten mathematischen Fläche einen Abstand aufweist, der größer als ein 0,2-faches des ersten maximalen Radius der ersten mathematischen Fläche ist. Weiter ist auch die zweite optische Linse eine zweite off-axis Linse, deren zweiter Flächenschwerpunkt ihrer zweiten Oberfläche von der zweiten Symmetrieachse der zweiten mathematischen Fläche einen Abstand aufweist, der größer als ein 0,2-faches des zweiten maximalen Radius der zweiten mathematischen Fläche ist. Alternativ ist der Abstand größer als 0,1% und insbesondere größer als 1% und weiter insbesondere größer als 5% des Radius des Kleinsten der durch die optisch wirksamen Oberflächen der jeweiligen off-axis Linse festgelegten Krümmungskreise. Dabei wird unter dem "Bereich des Abbildungsstrahlenganges" der Bereich einer jeweiligen optischen Oberfläche verstanden, der von einem jeweiligen Abbildungsstrahlenbündel ganz oder teilweise durchdrungen wird, welches Abbildungsstrahlenbündel von dem linken bzw. rechten Abbildungsstrahlengang geführt wird.

Ein maximaler Radius der jeweiligen rotationssymmetrischen mathematischen Fläche ist gemäß einer Ausführungsform durch die maximale Erstreckung derjenigen Linsenoberfläche bestimmt, die auch die mathematische Fläche festlegt.

Gemäß einer Ausführungsform ist die durch die Linsenoberfläche festgelegte rotationssymmetrische mathematische Fläche stetig. Gemäß einer weiteren Ausführungsform ist die rotationssymmetrische mathematische Fläche durchgehend konvex oder konkav und weist somit eine Krümmung mit gleichbleibendem Vorzeichen auf.

Die Berechnung des Flächenschwerpunktes ist dem Fachmann bekannt. Beispielhaft wird auf die Definition auf Seiten 57-59 des "Taschenbuch der Physik" von Horst Kuchling, 16. Auflage, Leipzig: Fachbuchverlag, 1996 verwiesen.

Das Vorsehen von off-axis Linsen in den ersten und zweiten optischen Teilsystemen erlaubt es bei geeigneter Wahl der optischen Oberflächen der off-axis Linsen sicherzustellen, daß sich von den beiden Abbildungsstrahlengängen der beiden Teilsysteme geführte Abbildungsstrahlenbündel auch nach einer Änderung eines Arbeitsabstands und/oder einer Abbildungsvergrößerung unter Einschluß eines von Null verschiedenen Stereowinkels α in einem Fokussierpunkt schneiden.

Weiter weisen die off-axis Linsen ein vergleichsweise niedriges Gewicht auf, da sie lediglich einen Abbildungsstrahlengang führen müssen und damit verglichen mit Linsen, die mehrere benachbarte Abbildungsstrahlengänge gleichzeitig führen, einen kleinen Durchmesser aufweisen.

Weiter erlaubt das separate Vorsehen von derartigen off-axis Linsen in den beiden optischen Teilsystemen zumindest in Bereichen (abschnittsweise) eine vollständig getrennte Führung der beiden linken und rechten Abbildungsstrahlengänge. Dies erlaubt eine optimale Faltung der von den beiden optischen Teilsystemen bereitgestellten Abbildungsstrahlengänge, so daß eine möglichst kleine Baulänge des stereoskopischen optischen System erzielt werden kann. Weiter verhindert eine getrennte Führung der Abbildungsstrahlengänge wechselseitige Störeinflüsse der von den Abbildungsstrahlengängen geführten Abbildungsstrahlenbündel beispielsweise aufgrund von Reflexionen.

Gemäß einer Ausführungsform, die mit der vorstehenden Ausführungsform kombiniert oder zu der vorstehenden Ausführungsform alternativ sein kann, sind die erste und zweite optische Linse des ersten bzw. zweiten Teilsystems jeweils off-axis Linsen und weisen jeweils wenigstens zwei optisch wirksame Flächen auf, die von einem Abbildungsstrahlengang nacheinander durchlaufen werden. Dabei können die off-axis Linsen auch als Kittglieder ausgebildet sein. Dann können die off-axis Linsen gemäß dieser Ausführungsform dadurch beschrieben sein, dass Verbindungsgeraden der Flächenschwerpunkte von wenigstens zwei optisch wirksamen Flächen einer jeweiligen off-axis Linse und Verbindungsgeraden der Krümmungsmittelpunkte (Mittelpunkte der durch die Oberflächen festgelegten Krümmungskreise) der wenigstens zwei optisch wirksamen Flächen einer jeweiligen off-axis Linse nicht co-axial, sondern voneinander beabstandet und/oder zueinander gewinkelt sind. Gemäß einer Ausführungsform beträgt der Abstand mehr als ein 0,2-faches eines maximalen Radius einer optisch wirksamen Fläche der off-axis Linse gemessen zwischen dem Flächenschwerpunkt und einem äußeren Rand der jeweiligen optisch wirksamen Fläche. Gemäß einer weiteren Ausführungsform durchsetzt die Verbindungsgerade der Krümmungsmittelpunkte die optisch wirksamen Flächen der jeweiligen off-axis Linse überhaupt nicht. Gemäß einer weiteren Ausführungsform durchsetzt die Verbindungsgerade der Krümmungsmittelpunkte der wenigstens zwei optisch wirksamen Flächen die optisch wirksamen Flächen der jeweiligen off-axis Linse in einem Abstand von dem Flächenschwerpunkt von wenigstens einer optisch wirksamen Fläche. Dabei beträgt der Abstand gemäß einer Ausführungsform mehr als ein 0,2-faches des maximalen Radius der optisch wirksamen Oberflächen der jeweiligen off-axis Linse und/oder mehr als 0,1% und insbesondere mehr als 1% und weiter insbesondere mehr als 5% des Radius des Kleinsten der durch die optisch wirksamen Oberflächen der off-axis Linse festgelegten Krümmungskreise.

Gemäß einer Ausführungsform ist der erste Flächenschwerpunkt der ersten Oberfläche der ersten off-axis Linse von der ersten Symmetrieachse gleichweit beabstandet, wie der zweite Flächenschwerpunkt der zweiten Oberfläche der zweiten off-axis Linse von der zweiten Symmetrieachse.

Diese Ausgestaltung der off-axis Linsen erlaubt auf besonders einfache Weise eine automatische Anpassung des Stereowinkels α, der von den in den beiden Abbildungsstrahlengängen der beiden Teilsysteme geführten Abbildungsstrahlenbündeln (in einem Fokussierpunkt) eingeschlossenen wird.

Weiter kann die erste Oberfläche der ersten off-axis Linse und die zweite Oberfläche der zweiten off-axis Linse im wesentlichen identisch sein. Dabei bedeutet im wesentlichen identisch, dass hinsichtlich wenigstens 90%, vorzugsweise wenigstens 98% und besonders bevorzugt 100% der ersten und zweiten Oberflächen der ersten und zweiten off-axis Linsen Übereinstimmung besteht, wobei nur optisch relevante Abweichungen betrachtet werden.

Dieser zumindest weitgehend identische Aufbau der off-axis Linsen erleichtert auch die Dimensionierung der übrigen optischen Elemente des stereoskopischen optischen Systems. Hierdurch können die Herstellungskosten des Systems gering gehalten werden. Weiter können die off-axis Linsen so beispielsweise einfach durch Aussägen aus einer großen Linse gewonnen werden.

Gemäß einer Ausführungsform weist eine dritte optische Linse des ersten optischen Teilsystems eine dritte optische Oberfläche auf, die im Bereich des linken Abbildungsstrahlengangs eine Teilfläche einer dritten rotationssymmetrischen mathematischen Fläche ist, welche dritte mathematische Fläche bezogen auf eine dritte Symmetrieachse einen dritten maximalen Radius aufweist. Weiter weist eine vierte optische Linse des zweiten optischen Teilsystems eine vierte optische Oberfläche auf, die im Bereich des rechten Abbildungsstrahlengangs eine Teilfläche einer vierten rotationssymmetrischen mathematischen Fläche ist, welche vierte mathematische Fläche bezogen auf eine vierte Symmetrieachse einen vierten maximalen Radius aufweist. Dabei ist die dritte optische Linse eine dritte off-axis Linse, deren dritter Flächenschwerpunkt ihrer dritten Oberfläche von der dritten Symmetrieachse der dritten mathematischen Fläche einen Abstand aufweist, der größer als ein 0,2-faches des dritten maximalen Radius der dritten mathematischen Fläche ist. Die vierte optische Linse ist eine vierte off-axis Linse, deren vierter Flächenschwerpunkt ihrer vierten Oberfläche von der vierten Symmetrieachse der vierten mathematischen Fläche einen Abstand aufweist, der größer als ein 0,2-faches des vierten maximalen Radius der vierten mathematischen Fläche ist. Weiter sind die erste off-axis Linse und die dritte off-axis Linse voneinander entlang des linken Abbildungsstrahlengangs um einen ersten Abstand sowie die zweite off-axis Linse und die vierte off-axis Linse voneinander entlang des rechten Abbildungsstrahlengangs um einen zweiten Abstand beabstandet. Dabei können der erste und der zweite Abstand wahlweise gleich oder voneinander verschieden sein.

Gemäß einer Ausführungsform, die mit der vorstehenden Ausführungsform kombiniert oder zu der vorstehenden Ausführungsform alternativ sein kann, sind auch die dritte und vierte optische Linse jeweils eine off-axis Linse. Auf die vorstehende alternative Beschreibung der off-axis Linsen anhand der Verbindungsgeraden der Flächenschwerpunkte von wenigstens zwei optisch wirksamen Flächen einer jeweiligen off-axis Linse und der Verbindungsgeraden der Krümmungsmittelpunkte der wenigstens zwei optisch wirksamen Flächen einer jeweiligen off-axis Linse wird Bezug genommen.

Gemäß einer Ausführungsform kann das stereoskopische optische System dann weiter wenigstens einen Aktuator umfassen, um den ersten Abstand zwischen der ersten off-axis Linse und der dritten off-axis Linse entlang des linken Abbildungsstrahlengangs und / oder den zweiten Abstand zwischen der zweiten off-axis Linse und der vierten off-axis Linse entlang des rechten Abbildungsstrahlengangs zu ändern.

Ein derartiger Aufbau ermöglicht eine Änderung des Arbeitsabstandes (Fokussierung) zur Anpassung an ein betrachtetes Objekt bei gleichzeitiger automatischer Anpassung eines Stereowinkels, ohne daß für den linken und rechten Strahlengang gemeinsame optische Elemente verwendet werden müssen. Die Änderung des Arbeitsabstandes erfolgt durch eine Veränderung des ersten bzw. zweiten Abstandes der ersten bzw. zweiten off-axis Linse relativ zu der dritten bzw. vierten off-axis Linse. Dabei stellt der erfindungsgemäße Aufbau bei geeigneter Wahl der optischen Oberflächen der off-axis Linsen sicher, daß sich von den beiden Abbildungsstrahlengängen der beiden Teilsysteme geführte Abbildungsstrahlenbündel auch nach einer Änderung eines Arbeitsabstands automatisch unter Einschluss eines von Null verschiedenen Stereowinkels α in einem Fokussierpunkt schneiden.

Gemäß einer Ausführungsform ist der Flächenschwerpunkt der Oberfläche der jeweiligen off-axis Linse von der jeweiligen Symmetrieachse um wenigstens 30% und bevorzugt um wenigstens 40% und besonders bevorzugt um wenigstens 50% des jeweiligen maximalen Radius beabstandet.

Bei einer Beabstandung um wenigstens 50% können wenigstens zwei off-axis Linsen durch Aussägen aus einer gemeinsamen großen Linse bereitgestellt werden.

Gemäß einer weiteren Ausführungsform können jeweils die erste und zweite off-axis Linse und/oder die dritte und vierte off-axis Linse bezogen auf eine gemeinsame Symmetrieebene paarweise symmetrisch angeordnet sein.

Ein derartiger symmetrischer Aufbau erleichtert die Konstruktion und Herstellung des stereoskopischen optischen Systems.

Weiter kann das erste optische Teilsystem wenigstens ein erstes Umlenkelement mit einer ersten Spiegelfläche aufweisen, das zur Faltung des linken Abbildungsstrahlengangs entlang des linken Abbildungsstrahlengangs zwischen einer Objektebene des Systems und der ersten off-axis Linse angeordnet ist. Entsprechend kann das zweite optische Teilsystem wenigstens ein zweites Umlenkelement mit einer zweiten Spiegelfläche aufweisen, das zur Faltung des rechten Abbildungsstrahlengangs entlang des rechten Abbildungsstrahlengangs zwischen einer Objektebene des Systems und der zweiten off-axis Linse angeordnet ist.

Aufgrund der Faltung weist das stereoskopische optische System eine im Vergleich zum Stand der Technik kompakte Baulänge auf. Dabei sind die erste und zweite off-axis Linse selber jeweils Teil der jeweiligen gefalteten Abbildungsstrahlengänge.

Gemäß einer Ausführungsform weist das erste optische Teilsystem weiter wenigstens eine erste Umlenkeinrichtung sowie ein drittes Umlenkelement mit einer dritten Spiegelfläche auf und weist das zweite optische Teilsystem weiter wenigstens eine zweite Umlenkeinrichtung sowie ein viertes Umlenkelement mit einer vierten Spiegelfläche auf. Dann ist die erste Umlenkeinrichtung im linken Abbildungsstrahlengang zwischen dem ersten Umlenkelement und dem dritten Umlenkelement angeordnet, und ist die zweite Umlenkeinrichtung im rechten Abbildungsstrahlengang zwischen dem zweiten Umlenkelement und dem vierten Umlenkelement angeordnet. Weiter ist die erste off-axis Linse zwischen dem ersten Umlenkelement und der ersten Umlenkeinrichtung angeordnet, und ist die zweite off-axis Linse zwischen dem zweiten Umlenkelement und der zweiten Umlenkeinrichtung angeordnet.

Somit können die Abbildungsstrahlengänge auch mehrfach gefaltet sein, wodurch die Kompaktheit des Systems erhöht wird.

Weiter kann das erste optische Teilsystem ein erstes Zoomsystem aufweisen, welches im linken Abbildungsstrahlengang zwischen dem dritten Umlenkelement und der ersten Umlenkeinrichtung angeordnet ist und relativ zueinander verlagerbare erste optische Zoom-Elemente aufweist, um eine variable Abbildungsvergrößerung des linken Abbildungsstrahlengangs zu bewirken. Dabei weist das zweite optische Teilsystem ein zweites Zoomsystem auf, welches im rechten Abbildungsstrahlengang zwischen dem vierten Umlenkelement und der zweiten Umlenkeinrichtung angeordnet ist und relativ zueinander verlagerbare zweite optische Zoom-Elemente aufweist, um eine variable Abbildungsvergrößerung des rechten Abbildungsstrahlengangs zu bewirken.

Durch die Anordnung der Zoom-Elemente in den jeweiligen gefalteten Strahlengängen kann eine variable Abbildungsvergrößerung bei minimaler Baulänge des stereoskopischen optischen Systems realisiert werden.

Gemäß einer Ausführungsform können die ersten optischen Zoom-Elemente des ersten Zoomsystems entlang einer gemeinsamen optischen Achse angeordnet sein, die mit der ersten Symmetrieachse der ersten off-axis Linse einen Winkel von maximal 20° einschließt. Weiter können auch die zweiten optischen Zoom-Elemente des zweiten Zoomsystems entlang einer gemeinsamen optischen Achse angeordnet sein, die mit der zweiten Symmetrieachse der zweiten off-axis Linse einen Winkel von maximal 20° einschließt.

Aufgrund der damit bewirkten starken Faltung der Abbildungsstrahlengänge wird die Baulänge des stereoskopischen optischen Systems gering gehalten. Zudem verlagert sich ein Schwerpunkt des Systems in Folge einer Verlagerung der Zoom-Elemente bei einer derartigen Anordnung nur geringfügig.

Weiter können die ersten optischen Zoom-Elemente des ersten Zoomsystems entlang einer gemeinsamen optischen Achse angeordnet sein, die zu der ersten Symmetrieachse der ersten off-axis Linse parallel versetzt ist. Dann können auch die zweiten optischen Zoom-Elemente des zweiten Zoomsystems entlang einer gemeinsamen optischen Achse angeordnet sein, die zu der zweiten Symmetrieachse der zweiten off-axis Linse parallel versetzt ist.

Gemäß einer weiteren Ausführungsform wird ein stereoskopisches optisches System offenbart, welches zusätzlich oder alternativ ein erstes optisches Teilsystem mit einer Mehrzahl von optischen Elementen zur Bereitstellung eines linken Abbildungsstrahlengangs des stereoskopischen optischen Systems umfasst, wobei das erste optische Teilsystem zur Faltung des linken Abbildungsstrahlengangs wenigstens ein Umlenkelement sowie eine erste Umlenkeinrichtung aufweist. Weiter weist das optische System ein zweites optisches Teilsystem mit einer Mehrzahl von optischen Elementen zur Bereitstellung eines rechten Abbildungsstrahlengangs des stereoskopischen optischen Systems auf, wobei das zweite optische Teilsystem zur Faltung des rechten Abbildungsstrahlengangs wenigstens ein Umlenkelement sowie eine zweite Umlenkeinrichtung aufweist. Dabei sind die optischen Elemente des ersten optischen Teilsystems und die optischen Elemente des zweiten optischen Teilsystems bezüglich einer gemeinsamen Symmetrieebene im wesentlichen symmetrisch angeordnet. Zudem liegen ein Hauptstrahl des linken Abbildungsstrahlengangs zwischen dem wenigstens einen Umlenkelement sowie der ersten Umlenkeinrichtung und ein Hauptstrahl des rechten Abbildungsstrahlengangs zwischen dem wenigstens einen Umlenkelement sowie der zweiten Umlenkeinrichtung jeweils in Ebenen, die mit der gemeinsamen Symmetrieebene jeweils einen kleinsten Winkel von zwischen 45° und 135° und bevorzugt zwischen 75° und 105° und besonders bevorzugt 90° einschließen. Weiter ist zwischen dem wenigstens einen Umlenkelement des linken Abbildungsstrahlengangs und der ersten Umlenkeinrichtung und/oder zwischen dem wenigstens einen Umlenkelement des rechten Abbildungsstrahlengangs und der zweiten Umlenkeinrichtung jeweils wenigstens eine optische Anordnung variabler Brechkraft angeordnet.

In diesem Zusammenhang bedeutet "im wesentlichen symmetrisch", dass die prinzipielle Anordnung der optischen Elemente symmetrisch ist, Asymmetrien im Kleinen (beispielsweise zur Dioptrienanpassung) jedoch zulässig sind. Insbesondere ist eine derartige Asymmetrie jedoch beispielsweise nicht größer als ein halber maximaler Durchmesser des größten verwendeten optischen Elements. Weiter wird unter einem Hauptstrahl der Strahl größter Intensität des von dem jeweiligen Abbildungsstrahlengang geführten Abbildungsstrahlenbündels verstanden. Zudem kann sich die optische Anordnung variabler Brechkraft aus einem oder mehreren optischen Elementen zusammensetzen.

Der vorstehend angegebene Winkelbereich stellt sicher, dass die Hauptstrahlen der in den jeweiligen Abbildungsstrahlengängen geführten Abbildungsstrahlenbündel im wesentlichen rechtwinklig gefaltet werden und nach der Faltung in Richtungen verlaufen, die im Wesentlichen quer zu einer Beobachtungsrichtung des stereoskopischen optischen Systems und damit in der Regel quer zu einer Stirn eines Benutzers ausgerichtet sind. Handelt es sich bei der optischen Anordnung variabler Brechkraft um eine verlagerbare optische Linse, so stellt die Anordnung von jeweils wenigstens einer verlagerbaren Linse in einem derart gefalteten linken und rechten Abbildungsstrahlengang sicher, dass ein Schwerpunkt des Systems bei einer gleichzeitigen entgegengesetzten Verlagerung beider Linsen beider Abbildungsstrahlengänge automatisch unverändert bleibt oder sich nur geringfügig ändert. Dies führt dazu, dass das System besonders gut für die Verwendung in Kopflupen geeignet ist. Der Grund ist, dass sich der am Kopf eines Benutzers durch das Tragen der Kopflupe angreifende Hebelarm auch bei einer Verlagerung der Linsen nicht oder nur wenig ändert. Bei Verwendung des optischen Systems in einem Operationsmikroskop hingegen wird beispielsweise die auf ein Stativ wirkende Kraft vergleichmäßigt.

Gemäß einer Ausführungsform schließt ein Hauptstrahl des linken Abbildungsstrahlengangs zwischen dem wenigstens einen Umlenkelement sowie der ersten Umlenkeinrichtung und ein Hauptstrahl des rechten Abbildungsstrahlengangs zwischen dem wenigstens einen Umlenkelement sowie der zweiten Umlenkeinrichtung mit der gemeinsamen Symmetrieebene jeweils einen kleinsten Winkel von zwischen 45° und 135° und bevorzugt zwischen 75° und 105° und besonders bevorzugt 90° ein. Dabei stellt der angegebene Winkelbereich sicher, dass die Hauptstrahlen der in den jeweiligen Abbildungsstrahlengängen geführten Abbildungsstrahlenbündel nach im wesentlichen rechtwinkliger Faltung in im wesentlichen entgegengesetzte Richtungen quer zu einer Beobachtungsrichtung des stereoskopischen optischen Systems und damit in der Regel quer zu einer Stirn eines Benutzers verlaufen.

In diesem Zusammenhang wird betont, dass der "kleinste Winkel" in einer beliebigen Ebene liegen kann, in welcher auch der jeweilige Hauptstrahl liegt.

Gemäß einer Ausführungsform weist das erste optische Teilsystem wenigstens ein erstes und ein drittes Umlenkelement auf. Weiter ist die erste Umlenkeinrichtung entlang des linken Abbildungsstrahlengangs zwischen den beiden ersten und dritten Umlenkelementen angeordnet. Dabei weist das zweite optische Teilsystem wenigstens ein zweites und ein viertes Umlenkelement auf, und ist die zweite Umlenkeinrichtung entlang des rechten Abbildungsstrahlengangs zwischen den beiden zweiten und vierten Umlenkelementen angeordnet. Zudem liegen jeweils Hauptstrahlen des linken Abbildungsstrahlengangs zwischen dem ersten bzw. dritten Umlenkelement und der ersten Umlenkeinrichtung sowie jeweils Hauptstrahlen des rechten Abbildungsstrahlengangs zwischen dem zweiten bzw. vierten Umlenkelement und der zweiten Umlenkeinrichtung jeweils in Ebenen, die mit der gemeinsamen Symmetrieebene jeweils einen kleinsten Winkel von zwischen 45° und 135° und bevorzugt zwischen 75° und 105° und besonders bevorzugt 90° einschließen.

Gemäß einer Ausführungsform schließen jeweils Hauptstrahlen des linken Abbildungsstrahlengangs zwischen dem ersten bzw. dritten Umlenkelement und der ersten Umlenkeinrichtung sowie jeweils Hauptstrahlen des rechten Abbildungsstrahlengangs zwischen dem zweiten bzw. vierten Umlenkelement und der zweiten Umlenkeinrichtung mit der gemeinsamen Symmetrieebene jeweils einen kleinsten Winkel von zwischen 45° und 135° und bevorzugt zwischen 75° und 105° und besonders bevorzugt 90° ein.

Diese wiederholte Faltung der Abbildungsstrahlengänge führt bei den angegebenen Winkelbereichen dazu, dass in den linken und rechten Abbildungsstrahlengängen geführte Hauptstrahlen in Bereichen des jeweiligen linken und rechten Abbildungsstrahlengangs im wesentlichen gegenparallel (d.h. im wesentlichen parallel und in entgegengesetzte Richtungen) verlaufen. Dies erhöht die Kompaktheit des Systems.

Weiter kann die wenigstens eine optische Anordnung variabler Brechkraft jeweils eine verlagerbare optische Linse eines ersten bzw. zweiten Zoomsystems sein, welches jeweils im linken bzw. rechten Abbildungsstrahlengang zwischen dem wenigstens jeweils einen Umlenkelement und der ersten bzw. zweiten Umlenkeinrichtung angeordnet ist. Das erste bzw. zweite Zoomsystem weist dabei jeweils relativ zueinander verlagerbare optische Zoom-Elemente auf, um eine variable Abbildungsvergrößerung des linken bzw. rechten Abbildungsstrahlengangs zu bewirken.

Die Linsen von Zoomsystemen müssen in der Praxis häufig verlagert werden, wobei die jeweiligen Linsen des linken und rechten Abbildungsstrahlenganges in der Regel zeitgleich und um die gleiche Distanz verlagert werden. Daher ist es von besonderem Vorteil, wenn ein Schwerpunkt des optischen Systems bei einer Verlagerung dieser Linsen im wesentlichen unverändert bleibt.

Dabei können die ersten optischen Zoom-Elemente des ersten Zoomsystems im linken Abbildungsstrahlengang entlang einer gemeinsamen optischen Achse angeordnet sein, die in einer Ebene liegt, welche mit der gemeinsamen Symmetrieachse oder Symmetrieebene einen kleinsten Winkel von zwischen 45° und 135° und bevorzugt zwischen 75° und 105° und besonders bevorzugt 90° einschließt. Entsprechend können die zweiten optischen Zoom-Elemente des zweiten Zoomsystems im rechten Abbildungsstrahlengang entlang einer gemeinsamen optischen Achse angeordnet sein, die in einer Ebene liegt, welche mit der gemeinsamen Symmetrieachse oder Symmetrieebene einen kleinsten Winkel von zwischen 45° und 135° und bevorzugt zwischen 75° und 105° und besonders bevorzugt 90° einschließt.

Aufgrund des angegebenen Winkelbereichs ist gewährleistet, dass ein Schwerpunkt des Systems sich auch bei einer Verlagerung von Linsen der Zoomsysteme nicht oder nur geringfügig verlagert.

Gemäß einer Ausführungsform schließen die gemeinsamen optischen Achsen der ersten bzw. zweiten optischen Zoom-Elemente des ersten bzw. zweiten Zoomsystems jeweils direkt mit der gemeinsamen Symmetrieachse oder Symmetrieebene einen kleinsten Winkel von zwischen 45° und 135° und bevorzugt zwischen 75° und 105° und besonders bevorzugt 90° ein.

Der angegebene Winkelbereich stellt sicher, dass die optischen Achsen der Zoomsysteme im wesentlichen senkrecht zu der gemeinsamen Symmetrieachse oder Symmetrieebene und in im wesentlichen entgegengesetzte Richtungen verlaufen. Gemäß einer Ausführungsform sind die ersten optischen Zoom-Elemente des ersten Zoomsystems zwischen dem dritten Umlenkelement und der ersten Umlenkeinrichtung entlang einer gemeinsamen optischen Achse angeordnet, die zu einem in dem linken Abbildungsstrahlengang zwischen dem ersten Umlenkelement und der ersten Umlenkeinrichtung geführten Hauptstrahl parallel versetzt ist. Weiter sind die zweiten optischen Zoom-Elemente des zweiten Zoomsystems zwischen dem vierten Umlenkelement und der zweiten Umlenkeinrichtung entlang einer gemeinsamen optischen Achse angeordnet, die zu einem in dem rechten Abbildungsstrahlengang zwischen dem zweiten Umlenkelement und der zweiten Umlenkeinrichtung geführten Hauptstrahl parallel versetzt ist.

Dies parallele Anordnung begünstigt eine hohe Kompaktheit des Systems.

Alternativ oder zusätzlich kann die zwischen dem wenigstens einen Umlenkelement des linken Abbildungsstrahlengangs und der ersten Umlenkeinrichtung und/oder zwischen dem wenigstens einen Umlenkelement des rechten Abbildungsstrahlengangs und der zweiten Umlenkeinrichtung jeweils vorgesehene wenigstens eine optische Anordnung variabler Brechkraft jeweils eine verlagerbare optische Linse sein. Die verlagerbare optische Linse ist jeweils zur Einstellung eines Arbeitsabstandes des stereoskopischen optischen Systems relativ zu einem weiteren optischen Element des linken bzw. rechten Abbildungsstrahlengangs verlagerbar.

Alternativ oder auch zusätzlich kann die wenigstens eine optische Anordnung variabler Brechkraft auch jeweils eine Flüssigkristalllinse oder eine Flüssigkeitslinse sein, deren jeweilige Brechkraft durch (vorzugsweise elektrisches) Ansteuern einstellbar ist.

Dabei kann bei geeigneter Wahl der optischen Oberflächen der wenigstens einen verlagerbaren optischen Linse bzw. Flüssigkristalllinse / Flüssigkeitslinse und des zugehörigen optischen Elements (beispielsweise eine optische Linse oder ein optischer Freiformspiegel (z. B. ein Spiegel mit nicht-torischer und nicht-spärischer (und somit atorischer) Spiegelfläche und rotationssymmetrischer Wirkung)), auf welches die Relativbewegung der optischen Linse bezogen ist, automatisch sichergestellt werden, dass sich die linken und rechten Abbildungsstrahlengänge auch nach einer Verlagerung unter Einschluss eines Stereowinkels in der Objektebene schneiden.

Weiter können die ersten und zweiten Umlenkeinrichtungen jeweils ausgebildet sein, um eine Faltung des linken bzw. rechten Abbildungsstrahlengangs um zwischen 155° und 205° und bevorzugt zwischen 170° und 190° und besonders bevorzugt 180° zu bewirken. Zudem können die ersten und zweiten und/oder die dritten und vierten Umlenkelemente jeweils ausgebildet sein, um eine Faltung des linken bzw. rechten Abbildungsstrahlengangs um zwischen 65° und 115° und bevorzugt zwischen 80° und 100° und besonders bevorzugt 90° zu bewirken.

Dies führt dazu, dass ein Bereich des linken und des rechten Abbildungsstrahlenganges jeweils U-förmig verläuft.

Dabei können die ersten und zweiten Umlenkeinrichtungen beispielsweise jeweils zwei optische Spiegelflächen aufweisen, die miteinander jeweils einen Winkel zwischen 85° und 95° und bevorzugt 90° einschließen.

Weiter können die erste und zweite Umlenkeinrichtung bezogen auf eine gemeinsame Symmetrieebene symmetrisch angeordnet sein.

Gemäß einer Ausführungsform kann ein Winkel zwischen der ersten Spiegelfläche des ersten Umlenkelements und der zweiten Spiegelfläche des zweiten Umlenkelements zwischen 50° und 130° und insbesondere zwischen 80° und 100° und bevorzugt 90° betragen.

Weiter können die ersten und zweiten Spiegelflächen des ersten und zweiten Umlenkelements bezogen auf eine gemeinsame Symmetrieebene symmetrisch angeordnet sein.

Das stereoskopische optische System kann weiter ein Bestrahlungssystem mit einer Strahlenquelle zum Emittieren von Strahlung und mit einer Bestrahlungs-Optik zum Bereitstellen eines Bestrahlungs-Strahlenganges für die von der Strahlenquelle emittierte Strahlung aufweisen.

Gemäß einer Ausführungsform können dann die ersten und zweiten Spiegelflächen des ersten und zweiten Umlenkelements jeweils zumindest bereichsweise für von dem Bestrahlungs-Strahlengang geführte Strahlung eine höhere Transparenz aufweisen als für im linken bzw. rechten Abbildungsstrahlengang geführte Strahlung und/oder voneinander um einen Abstand größer Null beabstandet sein. Dann verläuft der von der Bestrahlungs-Optik bereitgestellte Bestrahlungs-Strahlengang durch die erhöhte Transparenz der ersten und zweiten Spiegelflächen hindurch und/oder durch den Abstand zwischen den beiden ersten und zweiten Spiegelflächen hindurch.

In Verbindung mit einer Faltung der Abbildungsstrahlengänge kann so eine Null-Grad Bestrahlung (zum Beispiel eine Null-Grad Beleuchtung) realisiert werden. Auch eine Null-Grad Infrarotbestrahlung zum Beispiel für Fluoreszenzanwendung oder eine Null-Grad Laserbestrahlung zu Therapiezwecken ist so auf einfache Weise möglich.

Gemäß einer Ausführungsform weist wenigstens ein Umlenkelement und/oder wenigstens eine Umlenkeinrichtung des ersten und zweiten optischen Teilsystems jeweils eine gekrümmte Spiegelfläche auf, die einen Krümmungsradius kleiner als zehn Meter und vorzugsweise kleiner als ein Meter aufweist. Dabei kann die gekrümmte Spiegelfläche auch wenigstens zwei unterschiedliche Krümmungsradien aufweisen, wobei die wenigstens zwei Krümmungsradien kleiner als zehn Meter und vorzugsweise kleiner als ein Meter sind. Dabei beträgt ein Unterschied zwischen den wenigstens zwei unterschiedlichen (und vorzugsweise zueinander orthogonalen) Krümmungsradien mindestens 5% und bevorzugt mindestens 10% und besonders bevorzugt mindestens 20% des größeren Krümmungsradius der beiden Krümmungsradien. Die Spiegelflächen mit den vorsehend beschriebenen Krümmungsradien sind bevorzugt weder torisch noch sphärisch und weisen vorzugsweise eine von Null verschiedene Brennweite auf. Kennzeichnend ist, dass die entsprechend gekrümmten Spiegelflächen hinsichtlich des durch sie jeweils gefalteten Abbildungsstrahlengangs eine rotationssymmetrische Wirkung aufweisen.

Gemäß einer Ausführungsform sind die gekrümmten Spiegelflächen des wenigstens einen Umlenkelements und/oder der wenigstens einen Umlenkeinrichtung des ersten und zweiten optischen Teilsystems so ausgebildet und angeordnet, dass eine Verbindungsgerade zwischen einem Flächenschwerpunkt der gekrümmten Spiegelfläche des ersten Teilsystems und einem Flächenschwerpunkt der gekrümmten Spiegelfläche des zweiten Teilsystems, sowie eine Verbindungsgerade zwischen einem minimalen Krümmungsmittelpunkt (Mittelpunkt des durch die gekrümmte Spiegelfläche am Flächenschwerpunkt der gekrümmten Spiegelfläche festgelegten kleinsten Krümmungskreises) der gekrümmten Spiegelfläche des ersten Teilsystems und einem minimalen Krümmungsmittelpunkt der gekrümmten Spiegelfläche des zweiten Teilsystems, nicht co-axial sind. Beispielsweise können die Verbindungsgeraden der Flächenschwerpunkte und minimalen Krümmungsmittelpunkte der Spiegelflächen parallel versetzt, gewinkelt oder windschief zueinander sein. Gemäß einer Ausführungsform sind die Verbindungsgeraden der Flächenschwerpunkte und minimalen Krümmungsmittelpunkte der Spiegelflächen um mehr als 0,1% und insbesondere um mehr als 1% und weiter insbesondere um mehr als 5% eines minimalen Krümmungsradius am Flächenschwerpunkt von einer der gekrümmten Spiegelflächen beabstandet.

Somit ist es anstelle der vorstehend beschriebenen Verwendung von off-axis Linsen auch möglich, eine oder mehrere bzw. sogar alle off-axis Linsen (und benachbarte optische Umlenkelemente bzw. benachbarte Spiegelflächen von Umlenkeinrichtungen) durch gekrümmte optische Spiegel mit einer gekrümmten Spiegelfläche zu ersetzen. Dabei sind die gekrümmten Spiegelflächen jeweils so gewählt, dass eine Parallaxenfreiheit eines in dem jeweiligen Abbildungsstrahlengang geführten und von der jeweiligen Spiegelfläche reflektierten Abbildungsstrahlenbündels innerhalb eines Arbeitsbereichs des Systems immer bestehen bleibt. Die Verwendung gekrümmter Spiegel anstelle von off-axis Linsen und benachbarten optischen Umlenkelementen bzw. benachbarten Spiegelflächen von Umlenkeinrichtungen führt zu einer deutlichen Gewichtseinsparung des stereoskopischen optischen Systems. Dies ist insbesondere bei Verwendung des stereoskopischen optischen Systems als Kopflupe von Vorteil. Dabei können zwei entlang eines gemeinsamen Strahlengangs mit Abstand benachbarte gekrümmte Spiegel ebenso wie die vorstehend beschriebenen benachbarten off-axis Linsen mittels eines Aktuators relativ zueinander verlagert werden, um die Brennweite der so gebildeten optischen Anordnung zu verändern.

Gemäß einer Ausführungsform weist das erste optische Teilsystem weiter ein erstes Okularsystem und das zweite optische Teilsystem weiter ein zweites Okularsystem auf, wobei ein Hauptstrahl des in dem ersten Okularsystem geführten linken Abbildungsstrahlengangs sowie ein Hauptstrahl des in dem zweiten Okularsystem geführten rechten Abbildungsstrahlengangs im wesentlichen parallel zueinander verlaufen.

Dabei bedeutet "im wesentlichen parallel" dass sich zwei jeweils von einem der beiden Hauptstrahlen und einer kürzesten Verbindungsgeraden zwischen den beiden Hauptstrahlen, welche auf beiden Hauptstrahlen senkrecht steht, aufgespannte Ebenen (oder direkt Verlängerungen der beiden Strahlengänge) unter einem Winkel kleiner 20°, vorzugsweise kleiner 10° besonders bevorzugt kleiner 5° schneiden.

In Verbindung mit einem im wesentlichen symmetrischen Aufbau des optischen Systems führt diese Anordnung dazu, dass die Hauptstrahlen der in den beiden Okularsystemen geführten Abbildungsstrahlengänge auch im wesentlichen parallel zur gemeinsamen Symmetrieebene des optischen Systems verlaufen. Dabei können Hauptstrahlen des linken und rechten Abbildungsstrahlengangs im wesentlichen in einer gemeinsamen Ebene liegen.

Das stereoskopische optische System kann weiter ein Befestigungssystem aufweisen, welches konfiguriert ist, um an einem Kopf eines Benutzers befestigt zu werden.

Aufgrund des geringen Gewichts und des mit der kleinen Baulänge verbundenen geringen Hebelarms ist das erfindungsgemäße stereoskopische optische System besonders gut als Kopflupe geeignet.

Alternativ kann das stereoskopische optische System jedoch auch beispielsweise ein Stereomikroskop, insbesondere ein Operationsmikroskop, sein.

Im folgenden werden bevorzugte Ausführungsformen der vorliegenden Erfindung unter Bezugnahme auf die beigefügten Zeichnungen beschrieben. In den Zeichnungen sind gleiche oder ähnliche Elemente mit gleichen oder ähnlichen Bezugszeichen versehen. Dabei zeigt
- Figur 1: in schematischer Darstellung einen Strahlengang durch ein stereoskopisches optisches System gemäß einer ersten Aus- führungsform der vorliegenden Erfindung;
- Figur 2: in schematischer Darstellung eine Ansicht einer Kopflupe, in die das stereoskopische optische System aus Figur 1, Figur 4 oder Figur 5 in- tegriert ist;
- Figur 3A: eine schematische Aufsicht auf zwei off-axis Linsen vor dem Einbau in das stereoskopische optische System aus Figur 1, Figur 4 oder Figur 5;
- Figur 3B: eine schematische Aufsicht auf zwei alternative off-axis Linsen vor dem Einbau in das stereoskopische optische System aus Figur 1, Figur 4 oder Figur 5;
- Figur 3C: eine schematische Ansicht, welche wesentliche Eigenschaften von off- axis Linsen verdeutlicht;
- Figur 4: in schematischer Darstellung einen Strahlengang durch ein stereoskopisches optisches System gemäß einer ersten alternativen Ausführungsform der vorliegenden Erfindung;
- Figur 5: in schematischer Darstellung einen Strahlengang durch ein stereoskopisches optisches System gemäß einer zweiten alternativen Ausführungsform der vorliegenden Erfindung;
- Figur 6: in schematischer Darstellung einen Strahlengang durch ein aus dem Stand der Technik bekanntes Greenough-System; und
- Figur 7: in schematischer Darstellung einen Strahlengang durch ein weiteres stereoskopisches System nach dem Stand der Technik.

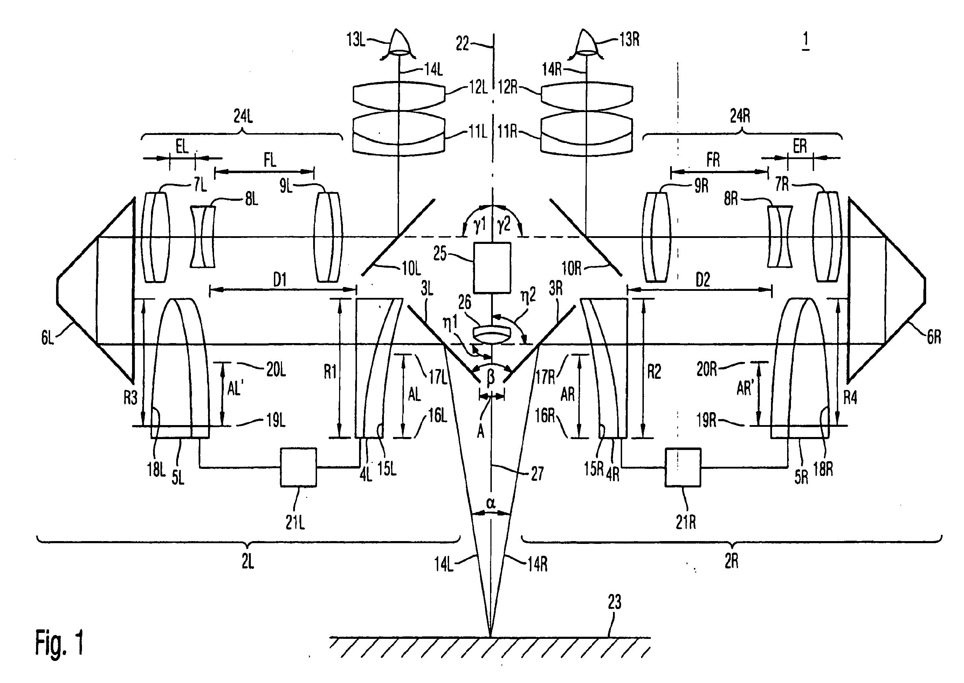

In Figur 1 ist schematisch ein Strahlengang durch ein stereoskopisches optisches System gemäß einer ersten Ausführungsform dargestellt.

Das stereoskopische optische System 1 ist für linke (erste) und rechte (zweite) Abbildungsstrahlengänge 14L und 14R bezüglich einer gemeinsamen Symmetrieebene 22 des optischen Systems 1 symmetrisch aus einem linken (ersten) optischen Teilsystem 2L und einem rechten (zweiten) optischen Teilsystem 2R aufgebaut. Zur Bereitstellung des linken und rechten Abbildungsstrahlengangs 14L, 14R weisen die linken und rechten optischen Teilsysteme 2L, 2R jeweils eine Mehrzahl optischer Elemente 3L, 3R, 4L, 4R, 5L, 5R, 6L, 6R, 7L, 7R, 8L, 8R, 9L, 9R, 10L, 10R, 11L, 11R, 12L, 12R auf. Dabei ist zu betonen, dass kleine Abweichungen von der Symmetrie beispielsweise zur Dioptrienanpassung jedoch zulässig sind. Vorzugsweise betragen diese Abweichungen jedoch nicht mehr als ein halber Durchmesser eines größten verwendeten optischen Elements.

In den Figuren 1 werden der linke und rechte Abbildungsstrahlengang 14L, 14R durch die jeweiligen Hauptstrahlen der sie durchlaufenden Abbildungsstrahlenbündel repräsentiert. Dabei ist ein Hauptstrahl der Strahl größter Intensität eines jeweiligen Strahlenbündels.

Ein linkes bzw. rechtes Auge 13L und 13R eines Benutzers blickt in jeweils ein linkes bzw. rechtes Austrittsokular des Systems 1. Das linke bzw. rechte Austrittsokular wird jeweils von zwei optischen Linsen 11L, 12L bzw. 11R, 12R, gebildet, die paarweise von einer (nicht gezeigten) Fassung getragen werden. Dabei verlaufen ein Hauptstrahl des in dem linken Austrittsokular geführten linken Abbildungsstrahlengangs 14L sowie ein Hauptstrahl des in dem rechten Austrittsokular geführten rechten Abbildungsstrahlengangs 14R relativ zueinander (und damit jeweils auch relativ zur gemeinsamen Symmetrieebene 22) parallel und in eine gleiche Richtung. Es ist jedoch keine absolute Parallelität erforderlich. Vielmehr ist es ausreichend, wenn sich zwei jeweils von einem der beiden Hauptstrahlen und einer kürzesten Verbindungsgeraden zwischen den beiden Hauptstrahlen (welche Verbindungsgerade auf beiden Hauptstrahlen senkrecht steht) aufgespannte Ebenen (oder direkt Verlängerungen der beiden Hauptstrahlen) unter einem Winkel kleiner 20°, vorzugsweise kleiner 10° besonders bevorzugt kleiner 5° schneiden. Alternativ zu dem linken bzw. rechten Austrittsokular kann auch jeweils ein (nicht gezeigter) Kameraadapter mit einem ortsauflösenden Digitalsensor vorgesehen sein.

Der linke und der rechte Abbildungsstrahlengang 14L und 14R werden durch Spiegelflächen von ersten, zweiten, dritten und vierten optischen Spiegeln 3L, 3R, 10L, 10R und Spiegelflächen eines ersten und zweiten Prismas 6L, 6R mehrfach gefaltet. Dabei ist das erste Prisma 6L zwischen dem ersten und dem dritten optischen Spiegel 3L, 10L angeordnet und bewirkt durch zwei aufeinanderfolgende Faltungen um 90° eine Faltung des linken Abbildungsstrahlenganges 14L um 180°. Entsprechend ist das zweite Prisma 6R zwischen dem zweiten und dem vierten optischen Spiegel 3R, 10R angeordnet und bewirkt durch zwei aufeinanderfolgende Faltungen um 90° eine Faltung des rechten Abbildungsstrahlenganges 14R um 180°.

Die Faltung durch das erste und zweite Prisma 6L, 6R kann alternativ aber auch von 180° verschieden sein und insbesondere zwischen 135° und 225° und bevorzugt zwischen 170° und 190° betragen.

Weiter schließt jeweils ein Hauptstrahl des linken Abbildungsstrahlengangs 14L zwischen dem ersten bzw. dritten optischen Spiegel 3L, 10L und dem ersten Prisma 6L sowie jeweils ein Hauptstrahl des rechten Abbildungsstrahlengangs 14R zwischen dem zweiten bzw. vierten optischen Spiegel 3R, 10R und dem zweiten Prisma 6R mit der gemeinsamen Symmetrieebene 22 jeweils einen kleinsten Winkel γ1, γ2, η1, η2 von jeweils 90° ein. Entsprechend bewirken der erste und zweite Spiegel 3L, 3R jeweils eine Faltung des linken bzw. rechten Abbildungsstrahlengangs 14L, 14R um im wesentlichen 90°. Dabei soll unter einer Faltung um "im wesentlichen 90°" eine Faltung um zwischen 65° und 115° und insbesondere eine Faltung um zwischen 80° und 100° verstanden werden.

Es ist zu betonen, dass die vorliegende Erfindung jedoch nicht auf einen Winkel γ1, γ2, η1, η2 von 90° beschränkt ist. Vielmehr kann der Winkel beispielsweise zwischen 45° und 135° und bevorzugt zwischen 75° und 105° betragen. Weiter ist es nicht zwingend erforderlich, dass die vorstehend genannten Winkel die allerkleinsten Winkel zwischen den jeweiligen Hauptstrahlen und der Symmetrieebene 22 sind. Vielmehr kann es ausreichend sein, wenn die Hauptstrahlen jeweils in Ebenen liegen, die mit der gemeinsamen Symmetrieebene 22 jeweils einen kleinsten Winkel γ1, γ2, η1, η2 von zwischen 45° und 135° und bevorzugt zwischen 75° und 105° und besonders bevorzugt 90° einschließen.

Weiter werden der linke und der rechte Abbildungsstrahlengang 14L und 14R durch erste, zweite, dritte und vierte optische Linsen 4L, 4R, 5L, 5R derart abgebildet, daß sie sich in einer Objektebene 23 schneiden und dabei einen von Null verschiedenen Stereowinkel α einschließen. Das stereoskopische optische System 1 bildet somit auf jedes Auge 13L, 13R eines Benutzers ein Bild eines in der Objektebene 23 angeordneten (nicht gezeigten) Objekts ab, wobei sich die Betrachtungswinkel der beiden Bilder um den Stereowinkel α unterscheiden. In der Folge entsteht beim Betrachter ein stereoskopischer Raumeindruck für das betrachtete Objekt. Die Größe des Stereowinkels α hängt von einem jeweiligen Arbeitsabstand der Objektebene von dem stereoskopischen optischen System 1 ab. Der Stereowinkel α beträgt vorzugsweise zwischen 1° und 18° und besonders bevorzugt zwischen 6° und 8°.

Von der Objektebene 23 kommend werden die beiden Abbildungsstrahlengänge 14L, 14R jeweils durch die Spiegelfläche des ersten und zweiten optischen Spiegels 3L bzw. 3R des linken bzw. rechten optischen Teilsystems 2L bzw. 2R um 90° in entgegengesetzte Richtungen gefaltet. In der gezeigten Ausführungsform schließen daher auch die jeweiligen Spiegelflächen des ersten und zweiten Spiegels 3L, 3R miteinander einen Winkel β von 90° ein. Weiter sind die Spiegelflächen des ersten und zweiten Spiegels 3L, 3R bezogen auf die gemeinsame Symmetrieebene 22 symmetrisch angeordnet.

Dabei ist der Winkel β nicht auf 90° beschränkt. Vielmehr können die jeweiligen Spiegelflächen des ersten und zweiten Spiegels 3L, 3R miteinander einen beliebigen Winkel β einschließen. Da für ein kompaktes optisches System 1 eine deutliche Faltung der beiden Abbildungsstrahlengänge 14L, 14R erforderlich ist, beträgt der Winkel β jedoch vorzugsweise zwischen 50° und 130°. Weiter ist die vorliegende Erfindung nicht auf die Verwendung von ersten bis vierten optischen Spiegeln 3L, 3R, 10L, 10R beschränkt. Vielmehr kann auch eine andere Anzahl von Spiegeln vorgesehen sein.

Hieraus wird deutlich, daß die beiden Abbildungsstrahlengänge 14L, 14R in den linken und rechten optischen Teilsystemen 2L, 2R vollständig getrennt geführt werden. Dies schließt jedoch nicht aus, daß zwischen der Objektebene 23 und den ersten und zweiten optischen Spiegeln 3L, 3R zusätzlich ein (in Figur 1 nicht gezeigtes) optisches Element wie beispielsweise eine Linse, ein Filter oder eine Abdeckscheibe vorgesehen ist, in welchem die beiden Abbildungsstrahlengänge 14L, 14R gemeinsam geführt werden.

Anschließend durchlaufen die so geführten Abbildungsstrahlengänge 14L, 14R getrennt erste und dritte bzw. zweite und vierte optische Linsen 4L, 5L bzw. 4R, 5R. Dabei sind die erste optische Linse 4L und die dritte optische Linse 5L voneinander entlang des linken Abbildungsstrahlengangs 14L um einen ersten Abstand D1 sowie die zweite optische Linse 4R und die vierte optische Linse 5R voneinander entlang des rechten Abbildungsstrahlengangs 14R um einen zweiten Abstand D2 beabstandet. Der erste optische Spiegel 3L ist somit zwischen der ersten optischen Linse 4L und der Objektebene 22 angeordnet und der zweite optische Spiegel 3R ist somit zwischen der zweiten optischen Linse 4R und der Objektebene 22 angeordnet. Dabei ist die erste optische Linse 4L eine erste off-axis Linse 4L.

Im Sinne der vorliegenden Erfindung wird unter off-axis Linse eine besondere Variante einer optischen Linse verstanden. Gemäß einer ersten Definition weist die off-axis Linse wenigstens im Bereich eines von dem jeweiligen Abbildungsstrahlengang geführten Abbildungsstrahlenbündels eine optische Oberfläche auf, die eine Teilfläche einer rotationssymmetrischen mathematischen Fläche ist, welche mathematische Fläche bezogen auf eine Symmetrieachse der mathematischen Fläche einen maximalen Radius aufweist. Der maximale Radius ist durch die maximale Erstreckung derjenigen optischen Oberfläche bestimmt, die auch die mathematische Fläche festlegt. Für eine off-axis Linse ist dabei kennzeichnend, daß ein jeweiliger Flächenschwerpunkt der optischen Oberfläche der Linse von der Symmetrieachse der zugehörigen mathematischen Fläche einen Abstand aufweist, der größer als ein 0,2-faches des maximalen Radius der mathematischen Fläche ist. Dieser Abstand kann beliebig größer sein und beispielsweise auch mehr als ein 0,3-faches oder mehr als ein 0,4-faches oder insbesondere mehr als ein 0,5-faches des maximalen Radius der mathematischen Fläche betragen. Weiter kann die Symmetrieachse der mathematischen Fläche innerhalb oder außerhalb der optischen Oberfläche der off-axis Linse liegen.

Übertragen auf die erste off-axis Linse 4L bedeutet dies, daß eine erste optische Oberfläche 15L der Linse 4L eine Teilfläche einer ersten rotationssymmetrischen mathematischen Fläche 31 ist. Ein erster Flächenschwerpunkt 17L dieser ersten optischen Oberfläche 15L der Linse 4L weist von einer ersten Symmetrieachse 16L der mathematischen Fläche 31 einen Abstand AL auf, der größer als ein 0,2-faches eines ersten maximalen Radius R1 der mathematischen Fläche 31 ist. In Figur 1 beträgt der Abstand AL ein 0,5-faches des ersten maximalen Radius R1.

Entsprechend sind auch die zweite, dritte und vierte optische Linse 4R, 5L und 5R off-axis Linsen und weisen wenigstens im Bereich eines von dem jeweiligen Abbildungsstrahlengang geführten Abbildungsstrahlenbündels jeweils eine zweite bzw. dritte bzw. vierte optische Oberfläche 15R bzw. 18L bzw. 18R auf, die jeweils eine Teilfläche einer zweiten bzw. dritten bzw. vierten rotationssymmetrischen mathematischen Fläche ist, welche mathematische Fläche bezogen auf eine zweite bzw. dritte bzw. vierte Symmetrieachse 16R bzw. 19L bzw. 19R der mathematischen Fläche jeweils einen zweiten bzw. dritten bzw. vierten maximalen Radius R2 bzw. R3 bzw. R4 aufweist. Dabei sind auch die zweite, dritte und vierte optische Linse 4R, 5L und 5R jeweils eine zweite, dritte und vierte off-axis Linse 4R, 5L und 5R. Folglich weist der jeweilige zweite bzw. dritte bzw. vierte Flächenschwerpunkt 17R bzw. 20L bzw. 20R der jeweiligen zweiten bzw. dritten bzw. vierten optischen Oberfläche 15R bzw. 18L bzw. 18R der zweiten bzw. dritten bzw. vierten off-axis Linse 4R bzw. 5L bzw. 5R von der jeweiligen zweiten bzw. dritten bzw. vierten Symmetrieachse 16R bzw. 19L bzw. 19R der jeweiligen mathematischen Fläche jeweils einen Abstand AR bzw. AL' bzw. AR' auf, der größer als ein 0,2-faches des jeweiligen zweiten bzw. dritten bzw. vierten maximalen Radius R2 bzw. R3 bzw. R4 der jeweiligen mathematischen Fläche ist. In Figur 1 beträgt der Abstand AR ein 0,5-faches des zweiten maximalen Radius R2 und betragen die Abstände AL' und AR' jeweils ein 0,4-faches des dritten bzw. vierten maximalen Radius R3 bzw. R4.

Der jeweilige Abstand AR bzw. AL' bzw. AR' zwischen dem jeweiligen zweiten bzw. dritten bzw. vierten Flächenschwerpunkt 17R bzw. 20L bzw. 20R und der jeweiligen zweiten bzw. dritten bzw. vierten Symmetrieachse 16R bzw. 19L bzw. 19R kann jeweils verschieden sein. In der vorliegenden Ausführungsform ist der erste Flächenschwerpunkt 17L der ersten Oberfläche 15L der ersten off-axis Linse 4L von der ersten Symmetrieachse 16L jedoch gleichweit beabstandet, wie der zweite Flächenschwerpunkt 17R der zweiten Oberfläche 15R der zweiten off-axis Linse 4R von der zweiten Symmetrieachse 16R. Entsprechend ist auch der dritte Flächenschwerpunkt 20L der dritten Oberfläche 18L der dritte off-axis Linse 5L von der dritten Symmetrieachse 19L gleichweit beabstandet, wie der vierte Flächenschwerpunkt 20R der vierten Oberfläche 18R der vierten off-axis Linse 5R von der vierten Symmetrieachse 19R.

In der gezeigten Ausführungsform sind die jeweiligen ersten, zweiten, dritten und vierten Flächenschwerpunkte 17L, 17R, 20L, 20R der ersten, zweiten, dritten und vierten Oberflächen 15L, 15R, 18L, 18R der jeweiligen ersten, zweiten, dritten und vierten off-axis Linse 4L, 4R, 5L, 5R von der jeweiligen ersten, zweiten, dritten und vierten Symmetrieachse 16L, 16R, 19L, 19R der zugehörigen mathematischen Fläche um 50% bzw. 40% des jeweiligen ersten, zweiten, dritten und vierten maximalen Radius R1, R2, R3, R4 der zugehörigen mathematischen Fläche beabstandet.

Die Erfindung ist hierauf jedoch nicht beschränkt. Der Abstand kann vielmehr auch kleiner sein. Eine Beabstandung von wenigstens 50% hat hingegen den Vorteil, daß wenigstens zwei off-axis Linsen durch Aussägen aus einer großen gemeinsamen Linse hergestellt werden können.

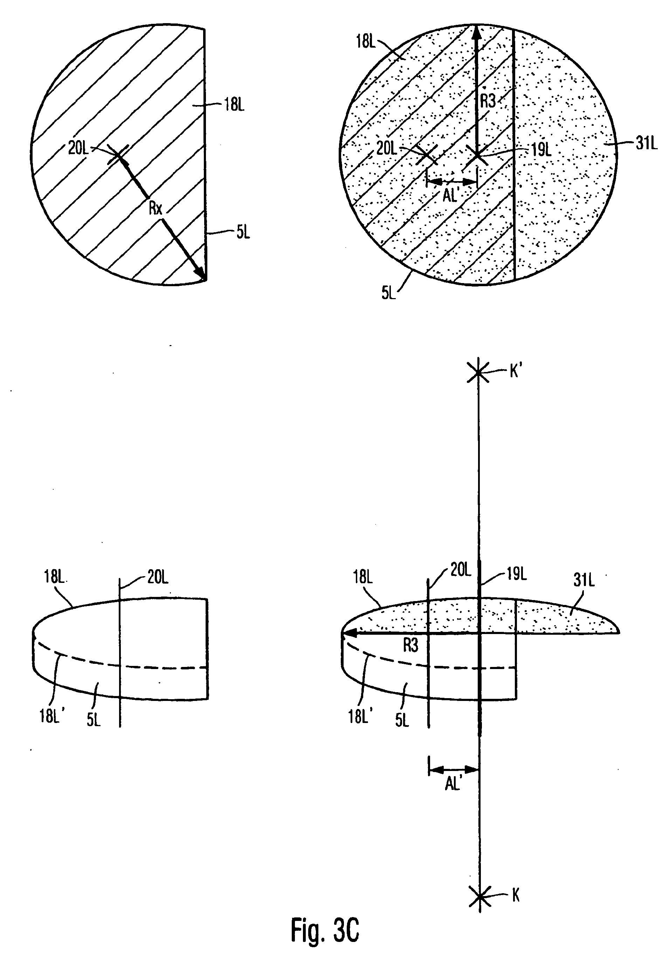

Der vorstehend beschriebene Zusammenhang zwischen der off-axis Linse und der durch die off-axis Linse beschriebenen rotationssymmetrischen mathematischen Fläche wird in Figur 3C am Beispiel der dritten off-axis Linse 5L verdeutlicht. Dabei zeigt Figur 3C in der linken Bildhälfte die dritte off-axis Linse 5L alleine und in der rechten Bildhälfte die dritte off-axis Linse 5L zusammen mit der durch diese festgelegten ersten rotationssymmetrischen mathematischen Fläche 31L. Dabei ist in Figur 3C oben jeweils eine Aufsicht und unten jeweils eine Schnittansicht der Linse bzw. Seitenansicht der Fläche gezeigt.

Die dritte optisch wirksame Oberfläche 18L der dritten off-axis Linse 5L legt eine (in der Figur gepunktete) rotationssymmetrische dritte mathematische Fläche 31L fest. In Figur 3C oben ist die dritte optische Oberfläche 18L diagonal schraffiert, und in Figur 3C unten wird sie durch die obere Kante der Schnittansicht der Linse 5L gezeigt. Wie in der rechten Hälfte von Figur 3C gezeigt, ist die dritte optische Oberfläche 18L der dritten off-axis Linse 5L eine Teilfläche der dritten mathematischen Fläche 31L. Weiter weist die dritte optische Oberfläche 18L der dritten off-axis Linse 5L einen Flächenschwerpunkt 20L auf. Die Berechnung des Schwerpunktes einer Fläche ist dem Fachmann bekannt.

Die durch die dritte optische Oberfläche 18L festgelegte dritte mathematische Fläche 31L weist einen auf ihre Symmetrieachse 19L bezogenen maximalen Radius R3 auf, der durch die maximale Erstreckung der dritten optischen Oberfläche 18L bestimmt wird, welche die mathematische Fläche 31L festlegt.

Ein Merkmal von off-axis Linsen ist, dass der Flächenschwerpunkt 20L der (dritten) optischen Oberfläche 18L von der Symmetrieachse 19L der durch die Linse festgelegten mathematischen Fläche 31L beabstandet ist.

Gemäß einer alternativen Ausführungsform können off-axis Linsen auch anders als vorstehend definiert werden. So weisen die off-axis Linsen 4L, 4R, 5L und 5R jeweils mindestens zwei optisch wirksame Oberflächen auf, die von einem Abbildungsstrahlengang 14L, 14R nacheinander durchsetzt werden. Diese optischen Oberflächen legen aufgrund ihrer Krümmung jeweils einen Mittelpunkt eines Krümmungskreises (Krümmungsmittelpunkt) fest. Dabei sind Verbindungsgeraden der Flächenschwerpunkte und Verbindungsgeraden der Krümmungsmittelpunkte dieser optisch wirksamen Oberflächen einer jeweiligen off-axis Linse anders als bei gewöhnlichen Linsen nicht co-axial, sondern parallel versetzt, gewinkelt oder windschief.

Dies wird in Figur 3C aufgrund des Abstandes AL' zwischen der Verbindungsgeraden der (in Figur 3C zusammenfallenden) Flächenschwerpunkte 20L und der (in Figur 3C mit der Symmetrieachse 19L der dritten mathematischen Fläche 31L zusammenfallenden) Verbindungsgerade der Krümmungsmittelpunkte K, K' der optisch wirksamen Flächen 18L, 18L' der dritten off-axis Linse 5L deutlich. Dabei bezeichnet K den Krümmungsmittelpunkt der Fläche 18L und K' den Krümmungsmittelpunkt der Fläche 18L'. In Figur 3C unten ist die optisch wirksame Fläche 18L' die gestrichelte mittlere Kante der Schnittansicht der Linse 5L. Gemäß einer Ausführungsform beträgt der Abstand mehr als ein 0,2-faches eines maximalen Radius einer optisch wirksamen Fläche der off-axis Linse 5L, gemessen zwischen dem Flächenschwerpunkt 20L und einem äußeren Rand der optisch wirksamen Flächen 18L und 18L'. Die vorliegende Erfindung ist hierauf jedoch nicht beschränkt. So kann der Abstand gemäß einer Ausführungsform mehr als 0,1% und insbesondere mehr als 1% und weiter insbesondere mehr als 5% des Radius des Kleinsten der durch die optisch wirksamen Oberflächen 18L, 18L' der off-axis Linse 5L festgelegten Krümmungskreise betragen.

In der gezeigten Ausführungsform sind die erste Oberfläche 15L der ersten off-axis Linse 4L und die zweite Oberfläche 15R der zweiten off-axis Linse 4R identisch. Ebenso sind die dritte Oberfläche 18L der dritten off-axis Linse 5L und die vierte Oberfläche 18R der vierten off-axis Linse 5R identisch. Diese völlige Übereinstimmung ist jedoch nicht zwingend erforderlich. Vielmehr kann es ausreichend sein, wenn hinsichtlich wenigstens 80%, vorzugsweise wenigstens 90% und besonders bevorzugt wenigstens 98% der jeweiligen Oberflächen der jeweiligen off-axis Linsen Übereinstimmung besteht, wobei nur optisch relevante Abweichungen betrachtet werden. Gemäß einer Ausführungsform wird nur paarweise Übereinstimmung von jeweils wenigstens einer Oberfläche unterschiedlicher off-axis Linsen gefordert.

Weiter sind die erste und zweite off-axis Linse 4L und 4R sowie die dritte und vierte off-axis Linse 5L und 5R bezogen auf die gemeinsame Symmetrieebene 22 jeweils paarweise symmetrisch angeordnet.

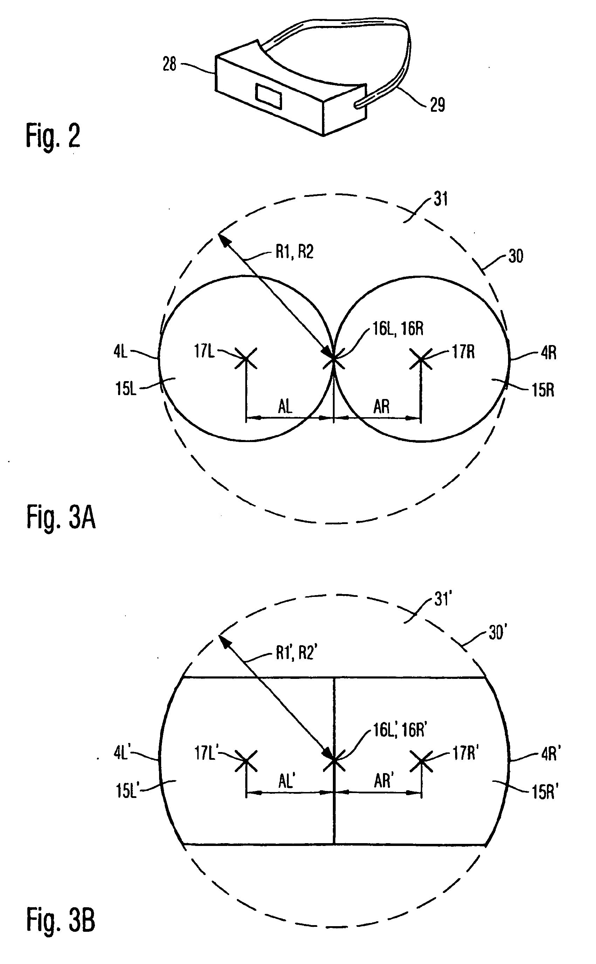

In Figur 3A ist eine schematische Aufsicht auf die erste und zweite off-axis Linse 4L, 4R vor dem Einbau in das stereoskopische optische System 1 gezeigt.

Dabei sind die erste und zweite off-axis Linse 4L, 4R durch Aussägen aus einer in gestrichelter Linie angedeuteten gemeinsamen rotationssymmetrischen optischen Linse 30 gebildet. Folglich sind die erste und zweite optische Oberfläche 15L, 15R der ersten und zweiten off-axis Linse 4L, 4R jeweils Teilflächen einer rotationssymmetrischen (mathematischen) Oberfläche 31 der gemeinsamen Linse 30, welche bezogen auf ihre Symmetrieachse 16L, 16R einen maximalen Radius R1, R2 aufweist. Somit sind in dieser besonderen Ausführungsform die erste und zweite Symmetrieachse 16L, 16R der gemeinsamen optischen Linse 30 sowie der erste und zweite maximale Radius R1, R2 der gemeinsamen optischen Linse 30 hinsichtlich der ersten und zweiten off-axis Linse 4L, 4R identisch. Ersichtlich weisen der erste Flächenschwerpunkt 17L der ersten Oberfläche 15L der ersten off-axis Linse 4L und der zweite Flächenschwerpunkt 17R der zweiten Oberfläche 15R der zweiten off-axis Linse 4R von der gemeinsamen Symmetrieachse 16L, 16R der gemeinsamen optischen Linse 30 jeweils einen Abstand AL, AR auf, der ein 0,5-faches des gemeinsamen maximalen Radius der gemeinsamen optischen Linse 30 R1, R2 beträgt.

Auch wenn die beiden in Figur 3A gezeigten ersten und zweiten off-axis Linsen 4L, 4R durch kreisförmiges Aussägen aus der gemeinsamen rotationssymmetrischen optischen Linse 30 gebildet sind, ist die vorliegende Erfindung hierauf nicht beschränkt. Alternativ können die beiden off-axis Linsen eine Umfangslinie beliebiger Form aufweisen. Insbesondere können die beiden off-axis Linsen auch unterschiedliche Umfangslinien aufweisen.

Besonders einfach können die beiden off-axis Linsen durch geradliniges Aussägen aus einer gemeinsamen rotationssymmetrischen optischen Linse 30' gebildet werden, wie es in Figur 3B schematisch gezeigt ist. In Figur 3B entsprechen die Elemente R1', R2', AL', AR', 4L', 4R', 15L', 15R', 16L', 16R', 17L', 17L', 30' und 31' den jeweiligen vorstehend beschriebenen Elementen R1, R2, AL, AR, 4L, 4R, 15L, 15R, 16L, 16R, 17L, 17L, 30 und 31 aus Figur 3A in lediglich zum Teil abgewandelter Form, so dass auf eine eigene Beschreibung verzichtet wird.

In der in Figur 1 gezeigten ersten Ausführungsform kann der erste Abstand D1 zwischen der ersten off-axis Linse 4L und der dritten off-axis Linse 5L entlang des linken Abbildungsstrahlengangs 14L mittels eines ersten Motors 21L verändert werden. Unter Verwendung eines zweiten Motors 21R kann der zweite Abstand D2 zwischen der zweiten off-axis Linse 4R und der vierten off-axis Linse 5R entlang des rechten Abbildungsstrahlengangs 14R geändert werden. Dabei bilden die jeweils in Reihe entlag des Abbildungsstrahlenganges 14L bzw. 14R angeordnete erste und dritte bzw. zweite und vierte off-axis Linse 4L, 4R, 5L, 5R paarweise eine optische Anordnung variabler Brechkraft. Die so gebildete optische Anordnung variabler Brechkraft ist jeweils zwischen dem ersten optischen Spiegel 3L und dem ersten Prisma 6L sowie dem zweiten optischen Spiegel 3R und dem zweiten Prisma 6R angeordnet. Dabei handelt es sich bei den ersten und zweiten Motoren 21L und 21R jeweils um Schrittmotoren. Anstelle von separaten Schrittmotoren kann beispielsweise auch ein gemeinsamer Motor oder ein oder zwei manuelle Antriebe verwendet werden.

Bei dem in der Figur 1 gezeigten symmetrischen Aufbau des stereoskopischen optischen Systems 1 ist es vorteilhaft, wenn eine relative Verlagerung der ersten off-axis Linse 4L zu der dritten off-axis Linse 5L und eine relative Verlagerung der zweiten off-axis Linse 4R zu der vierten off-axis Linse 5R mechanisch oder elektrisch so gekoppelt sind, daß der erste Abstand D1 und der zweite Abstand D2 immer gleich groß sind. Dies kann bei der Verwendung von zwei Schrittmotoren beispielsweise durch eine gemeinsame Ansteuerung mittels einer in Figur 1 nicht gezeigten Steuereinrichtung erfolgen. Bei der Verwendung eines gemeinsamen Motors oder eines gemeinsamen manuellen Antriebs kann dies beispielsweise durch eine mechanische Kopplung erfolgen.

Durch Veränderung der Abstände D1 und D2 wird eine Anpassung des stereoskopischen optischen Systems 1 an einen jeweiligen Arbeitsabstand der Objektebene 23 und damit eine Fokussierung ermöglicht. Dabei stellt der erfindungsgemäße Aufbau bei geeigneter Wahl der für die erste und dritte bzw. für die zweite und vierte off-axis Linse 4L, 5L bzw. 4R, 5R jeweils verwendeten ersten und dritten bzw. zweiten und vierten optischen Oberflächen 15L, 18L bzw. 15R, 18R sicher, daß von Hauptstrahlen des linken und rechten Abbildungsstrahlenganges 14L, 14R in der Objektebene 23 auch nach der Änderung des Arbeitsabstandes und damit nach einer Änderung der Abstände D1 und D2 innerhalb eines Arbeitsbereichs des Systems 1 automatisch immer ein von Null verschiedener Stereowinkel α eingeschlossen wird.

Nach dem Durchlaufen der ersten und dritten bzw. zweiten und vierten off-axis Linsen 4L, 5L bzw. 4R und 5R treten die getrennt geführten linken und rechten Abbildungsstrahlengänge 14L und 14R jeweils in die ersten und zweiten Prismen (Umlenkeinrichtungen) 6L und 6R ein, welche jeweils zwei Spiegelflächen aufweisen. Somit sind die erste und dritte off-axis Linse 4L, 5L zwischen dem ersten Spiegel 3L und dem ersten Prisma 6L angeordnet. Entsprechend sind die zweite und vierte off-axis Linse 4R, 5R zwischen dem zweiten Spiegel 3R und dem zweiten Prisma 6R angeordnet.

Dabei können die beiden Prismen 6L und 6R, welche die Umlenkeinrichtungen bildenden, wahlweise auch durch Spiegelpaare ersetzt sein.

Nach der Faltung durch das erste und zweite Prisma 6L, 6R treten die getrennt geführten linken und rechten Abbildungsstrahlengänge 14L, 14R in ein erstes bzw. zweites Zoomsystem 24L, 24R des linken bzw. rechte optischen Teilsystems 2L, 2R ein.

Das erste Zoomsystem 24L weist drei relativ zueinander verlagerbare optische Linsen (erste optische Zoom-Elemente) 7L, 8L, 9L auf, um eine variable Abbildungsvergrößerung des linken Abbildungsstrahlengangs 14L zu bewirken und so eine optische Anordnung variabler Brechkraft zu bilden. Entsprechend weist das zweite Zoomsystem 24R drei relativ zueinander verlagerbare optische Linsen (zweite optische Zoom-Elemente) 7R, 8R, 9R auf, um eine variable Abbildungsvergrößerung des rechten Abbildungsstrahlengangs 14R zu bewirken und so eine optische Anordnung variabler Brechkraft zu bilden. Somit ist zwischen dem ersten Prisma 6L und dem dritten optischen Spiegel 10L sowie dem zweiten Prisma 6R und dem vierten optischen Spiegel 10R jeweils wenigstens eine optische Anordnung variabler Brechkraft angeordnet.

Dabei sind die optischen Linsen 7L, 8L, 9L des ersten Zoomsystems 24L entlang einer gemeinsamen optischen Achse angeordnet, die zu der ersten Symmetrieachse 16L der ersten off-axis Linse 4L parallel versetzt ist, und die mit der gemeinsamen Symmetrieebene / Symmetrieachse 22 des stereoskopischen optischen Systems 1 einen kleinsten Winkel von 90° einschließt. Entsprechend sind auch die optischen Linsen 7R, 8R, 9R des zweiten Zoomsystems 24R entlang einer gemeinsamen optischen Achse angeordnet, die zu der zweiten Symmetrieachse 16R der zweiten off-axis Linse 4R parallel versetzt ist, und die mit der gemeinsamen Symmetrieebene / Symmetrieachse 22 des stereoskopischen optischen Systems 1 einen kleinsten Winkel von 90° einschließt.

Folglich sind die jeweiligen optischen Achse der optischen Linsen 7L, 8L, 9L, 7R, 8R, 9R zu einem zwischen dem ersten optischen Spiegel 3L und dem ersten Prisma 6L bzw. dem zweiten optischen Spiegel 3R und dem zweiten Prisma 6R jeweils geführten Hauptstrahl parallel versetzt.

Die vorliegende Erfindung ist jedoch nicht auf eine derartige Anordnung der optischen Linsen 7L, 8L, 9L, 7R, 8R, 9R des ersten und zweiten Zoomsystems 24L, 24R beschränkt. Vielmehr können die jeweiligen optischen Achsen der optischen Linsen 7L, 8L, 9L, 7R, 8R, 9R des ersten und zweiten Zoomsystems 24L, 24R mit der ersten bzw. zweiten Symmetrieachse 16L bzw. 16R der ersten bzw. zweiten off-axis Linse 4L bzw. 4R auch jeweils einen Winkel einschließen. Um eine ausreichende Faltung des Strahlenganges und damit eine kleine Bauform zu erzielen, beträgt der eingeschlossene Winkel jeweils vorzugsweise maximal 20°. Entsprechend ist der von der jeweiligen optischen Achse der optischen Linsen 7L, 8L, 9L, 7R, 8R, 9R und der gemeinsamen Symmetrieebene 22 des stereoskopischen optischen Systems 1 jeweils eingeschlossene kleinste Winkel auch nicht auf 90° beschränkt. Vielmehr kann dieser kleinste Winkel beispielsweise zwischen 45° und 135° und insbesondere zwischen 75° und 105° betragen. Dabei kann dieser kleinste Winkel in einer beliebigen Ebene um die jeweilige optische Achse herum angeordnet sein.

Durch Änderung von Linsenabständen EL, ER und FL, FR zwischen den optischen Linsen 7L, 8L, 9L, 7R, 8R, 9R des ersten und zweiten Zoomsystems 24L, 24R ist eine Änderung der Brechkraft des jeweiligen ersten und zweiten Zoomsystems 24L, 24R und so eine einstellbare Vergrößerung (Zoomfunktion) der von den linken und rechten Abbildungsstrahlengängen 14L, 14R jeweils bewirkten Abbildung möglich.

Dies erfolgt in der gezeigten Ausführungsform durch Verlagerung der optischen Linsen 7L, 8L, 9L des ersten Zoomsystems 24L bzw. der optischen Linsen 7R, 8R, 9R des zweiten Zoomsystems 24R relativ zueinander mittels wenigstens einer (nicht gezeigten) Stelleinrichtung. Dabei erfolgt die Verlagerung bei dem in Figur 1 gezeigten symmetrischen Aufbau des stereoskopischen optischen Systems 1 bevorzugt so, daß die Abstände EL und ER sowie die Abstände FL und FR immer gleich groß sind. Die beschriebene Anordnung der optischen Linsen 7L, 8L, 9L, 7R, 8R, 9R des ersten und zweiten Zoomsystems 24L, 24R führt dazu, dass sich ein Schwerpunkt des stereoskopischen optischen Systems 1 bei einer gleichzeitigen Verlagerung von Linsen des ersten und zweiten Zoomsystems 24L, 24R nicht oder nur geringfügig ändert.

Gemäß einer (nicht eigens gezeigten) alternativen Ausführungsform wird die Änderung der Brechkraft des jeweiligen ersten und zweiten Zoomsystems 24L, 24R nicht durch relative Verlagerung der optischen Linsen 7L, 8L, 9L des ersten Zoomsystems 24L bzw. der optischen Linsen 7R, 8R, 9R des zweiten Zoomsystems 24R bewirkt. Vielmehr ist gemäß dieser alternativen Ausführungsform jeweils wenigstens eine der optischen Linsen 7L, 8L, 9L des ersten Zoomsystems 24L bzw. der optischen Linsen 7R, 8R, 9R des zweiten Zoomsystems 24R eine optische Linse variabler Brechkraft, wobei die Brechkraft durch Ansteuern der jeweiligen optischen Linse veränderbar ist.

Gemäß einer (nicht eigens gezeigten) weiteren alternativen Ausführungsform erfolgt eine Änderung der Brechkraft der durch die Linsen 4L und 5L bzw. 4R und 5R jeweils gebildeten optischen Anordnung zur Anpassung des Arbeitsabstandes zusätzlich oder alternativ nicht durch relative Verlagerung einer der optischen Linsen 4L, 5L, 4R, 5R. Vielmehr ist gemäß dieser weiteren alternativen Ausführungsform jeweils wenigstens eine der optischen Linsen 4L, 5L, 4R, 5R eine optische Linse variabler Brechkraft, oder ist zwischen den Paaren der off-axis Linsen jeweils eine optische Linse variabler Brechkraft angeordnet, wobei die Brechkraft durch Ansteuern der jeweiligen optischen Linse veränderbar ist.

Optische Linsen mit variabler und damit einstellbarer und änderbarer Brechkraft sind aus dem Stand der Technik, beispielsweise aus US 4,795,248 oder US 5,815,233, deren Offenbarung in die vorliegende Anmeldung durch Inbezugnahme vollumfänglich aufgenommen wird, bekannt. Solche Linsen mit einstellbarer Brechkraft umfassen eine Flüssigkristallschicht, welche über eine Elektrodenstruktur ansteuerbar ist, um eine durch die Flüssigkristallschicht bereitgestellte optische Weglänge für einen die Schicht durchsetzenden Strahl ortsabhängig, das heißt über den Querschnitt der Linse unterschiedlich, auf gewünschte Werte einzustellen, wodurch eine flexible Linsenwirkung erreichbar ist. Derartige Linsen werden als Flüssigkristalllinsen oder LCD-Linsen bezeichnet.

Alternativ kann es sich bei einer derartigen optischen Linse variabler Brechkraft beispielsweise auch um eine Flüssigkeitslinse handeln. Eine Flüssigkeitslinse umfaßt typischerweise ein Gehäuse mit zwei Eintritts- bzw. Austrittsfenstern, zwischen welchen zwei vorzugsweise nicht wesentlich miteinander mischbare Flüssigkeiten mit unterschiedlichem Brechungsindex eingeschlossen sind. Das Gehäuse stellt für die beiden Flüssigkeiten eine bezüglich einer optischen Achse der Flüssigkeitslinse symmetrische konische Wand bereit, an der eine Grenzfläche zwischen den beiden Flüssigkeiten unter einem Kontaktwinkel anliegt. Eine Flüssigkeit ist elektrisch leitend, während die andere Flüssigkeit elektrisch im wesentlichen nichtleitend ist. Durch Anlegen einer Spannung kann der Winkel, den die Grenzfläche zwischen den beiden Flüssigkeiten mit der Wand einschließt, geändert werden. Aufgrund der unterschiedlichen Brechungsindizes der beiden Flüssigkeiten ist damit eine Linsenwirkung der Linse für einen diese entlang der optischen Achse durchsetzenden Strahl änderbar. Eine Flüssigkeitslinse kann beispielsweise von der Firma Varioptic, 69007 Lyon, Frankreich, bezogen werden. Weitere Flüssigkeitslinsen, welche zur Änderung ihrer Brechkraft eine Änderung einer Form einer Grenzfläche ausnutzen, sind aus US 6,369,954 B1, CA 2,368,553 und US 4,783,155 bekannt, deren Offenbarung in die vorliegende Anmeldung durch Inbezugnahme vollumfänglich aufgenommen wird.

Nach dem Durchlaufen des ersten und zweiten Zoomsystems 24L, 24R werden die getrennt geführten linken und rechten Abbildungsstrahlengänge 14L, 14R durch die Spiegelfläche des im linken optischen Teilsystem 2L angeordneten dritten optischen Spiegels (dritten Umlenkelements) 10L bzw. durch die Spiegelfläche des im rechten optischen Teilsystem 2R angeordneten vierten optischen Spiegels (vierten Umlenkelements) 10R gefaltet.

Das erste Zoomsystem 24L des ersten optischen Teilsystems 2L ist somit zwischen dem dritten optischen Spiegel 10L und dem ersten Prisma 6L angeordnet, und das erste Prima 6L ist zwischen dem ersten optischen Spiegel 3L und dem dritten optischen Spiegel 10L angeordnet. Entsprechend ist das zweite Zoomsystem 24R des zweiten optischen Teilsystems 2R zwischen dem vierten optischen Spiegel 10R und dem zweiten Prisma 6R angeordnet, und das zweite Prima 6R ist zwischen dem zweiten optischen Spiegel 3R und dem vierten Spiegel 10R angeordnet.

Das in Figur 1 gezeigte stereoskopische optische System 1 weist gemäß der ersten Ausführungsform weiter ein durch eine Strahlenquelle 25 und eine Bestrahlungs-Optik 26 gebildetes Bestrahlungssystem auf. Die Strahlenquelle 25 dient zum Emittieren von Strahlung. Die Bestrahlungs-Optik 26, welche in Figur 1 nur eine einzige Linse besitzt, stellt einen Bestrahlungs-Strahlengang 27 für die von der Strahlenquelle 25 emittierte Strahlung bereit. Bei der von der Strahlenquelle 25 emittierten Strahlung kann es sich beispielsweise um zu Beleuchtungszwecken emittiertes Licht, zu Therapiezwecken emittierte Laserstrahlung und/oder zu Diagnosezwecken emittierte Infrarotstrahlung handeln. Alternativ zum Bestrahlungssystem kann auch ein Beobachtungssystem, insbesondere ein Infrarot-Beobachtungssystem, vorgesehen sein.

Wie aus Figur 1 gut ersichtlich, sind die ersten und zweiten Spiegelflächen der ersten und zweiten Spiegel 3L, 3R des ersten und zweiten optischen Teilsystems 2L, 2R voneinander um einen Abstand A größer Null beabstandet, und ist der von der Bestrahlungs-Optik 26 bereitgestellte Bestrahlungs-Strahlengang 27 durch den Abstand A hindurch geführt. Auf diese Weise wird eine Null-Grad Bestrahlung/Beobachtung eines in der Objektebene 22 angeordneten Objektes ohne weiteres ermöglicht.

Alternativ zu dem in Figur 1 gezeigten Abstand A zwischen der ersten und der zweiten Spiegelfläche des ersten und des zweiten Spiegels 3L, 3R können die ersten und zweiten Spiegelflächen des ersten und zweiten Spiegels 3L, 3R jeweils zumindest bereichsweise eine höhere Transparenz für von dem Bestrahlungs-Strahlengang 27 geführte Strahlung aufweisen, als für im linken bzw. rechten Abbildungsstrahlengang 14L, 14R geführte Abbildungsstrahlenbündel. Somit können die Spiegelflächen der ersten und zweiten Spiegel 3L, 3R bereichsweise transparent oder semi-transparent sein oder eine dichroitische Eigenschaft aufweisen. In diesem Fall ist der von der Bestrahlungs-Optik 26 bereitgestellte Bestrahlungs-Strahlengang 27 durch den Bereich erhöhter Transparenz bzw. dichroitischer Eigenschaft der ersten und zweiten Spiegelflächen hindurch geführt.

In der in Figur 1 gezeigten Ausführungsform liegen Hauptstrahlen der von dem linken und dem rechten Abbildungsstrahlengang 14L, 14R jeweils geführten linken und rechten Abbildungsstrahlenbündel im wesentlichen in einer gemeinsamen Ebene. Dies bedeutet, dass die Hauptstrahlen mit einer gemeinsamen Ebene einen Winkel kleiner 30° und insbesondere kleiner 20° und insbesondere kleiner 10° und vorzugsweise 0° einschließen.

Es wird betont, daß die vorstehend beschriebene erste Ausführungsform nur beispielhaft ist. So kann insbesondere von der in Figur 1 gezeigten Anzahl der optischen Elemente abgewichen werden. Weiter können eine oder mehrere der verwendeten optischen Linsen (wie gezeigt) Kittglieder sein. Zudem können anstelle von optischen Spiegeln beliebige andere optische Umlenkelemente wie beispielsweise Prismen verwendet werden.

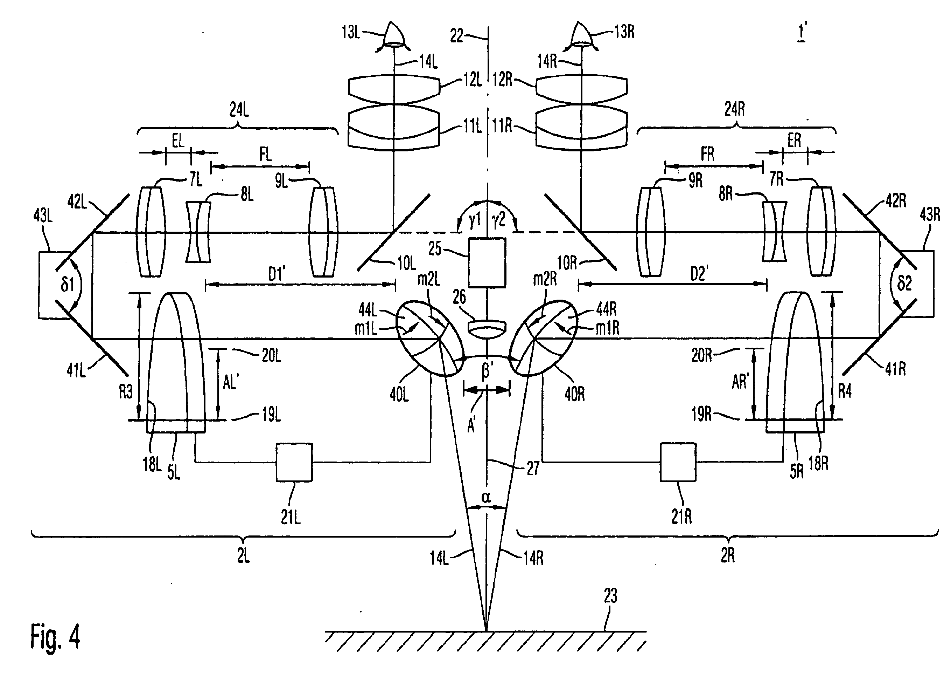

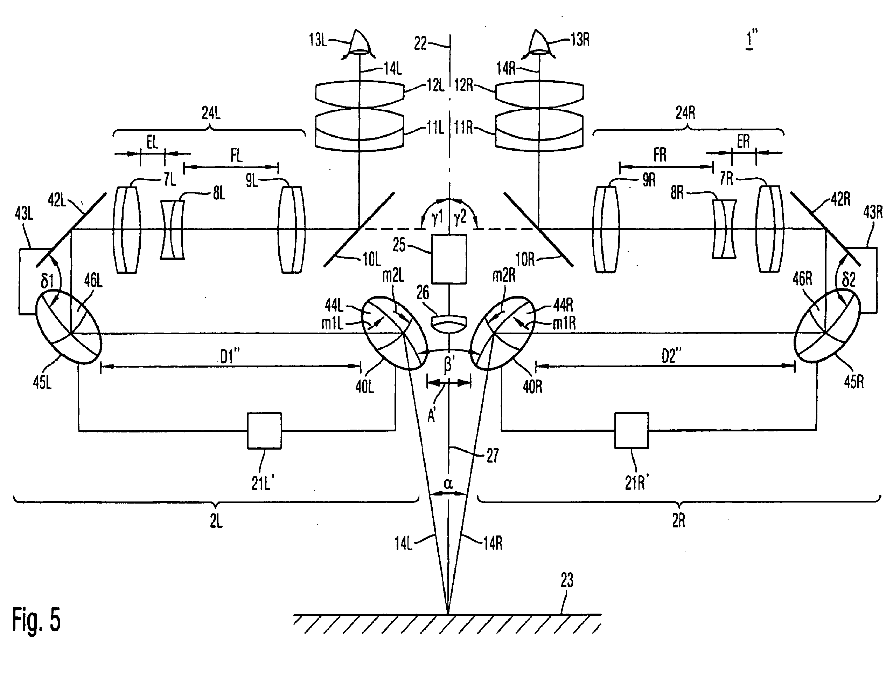

In den Figuren 4 und 5 sind in schematischer Darstellung jeweils Strahlengänge durch ein stereoskopisches optisches System gemäß zweier alternativer Ausführungsformen gezeigt.

Da der Aufbau der Strahlengänge der beiden alternativen Ausführungsformen eine große Ähnlichkeit zu dem in Figur 1 gezeigten und vorstehend beschriebenen Strahlengang gemäß der ersten Ausführungsform aufweist, wird im Folgenden nur auf die Unterschiede eingegangen. So sind in den Figuren 4 und 5 beispielsweise die Winkel η1, η2 der besseren Übersichtlichkeit halber (anders als in Figur 1) nicht eigens gezeigt.

Das stereoskopische optische System 1' gemäß der in Figur 4 gezeigten ersten alternativen Ausführungsform unterscheidet sich von dem stereoskopischen optischen System 1 gemäß der vorstehend beschriebenen ersten Ausführungsform insbesondere dadurch, dass die erste und zweite off-axis Linse 4L, 4R und die den off-axis Linsen 4L, 4R jeweils benachbarten ersten und zweiten optischen Spiegel 3L und 3R jeweils paarweise in dem linken und rechten Abbildungsstrahlengang 14L und 14R durch erste und zweite gekrümmte optische Spiegel 40L, 40R ersetzt sind.

Dabei beträgt ein (in Figur 4 jeweils durch Pfeile symbolisierter) größter Krümmungsradius m1L, m1R der gekrümmten Spiegelflächen 44L, 44R der ersten und zweiten gekrümmten optischen Spiegel 40L, 40R jeweils 80cm und ist somit kleiner als ein Meter. Die vorliegende Anmeldung ist hierauf jedoch nicht beschränkt. Vielmehr kann es für die Zwecke dieser Anmeldung ausreichend sein, wenn der jeweilige größte Krümmungsradius m1L, m1R der gekrümmten Spiegelflächen 44L, 44R kleiner als zehn Meter ist.

In der gezeigten Ausführungsform weisen die gekrümmten Spiegelflächen 44L, 44R jeweils zwei unterschiedliche Krümmungsradien m1L, m2L bzw. m1R, m2R auf, wobei ein Unterschied zwischen den unterschiedlichen, vorzugsweise zueinander orthogonalen Krümmungsradien m1L und m2L bzw. m1R und m2R jeweils 22% des größeren Krümmungsradius m1R bzw. m1L der jeweils zwei Krümmungsradien m1L, m2L bzw. m1R, m2R einer jeweiligen gekrümmten Spiegelflächen 44L, 44R beträgt.

Die vorliegende Erfindung ist jedoch nicht darauf beschränkt, dass die gekrümmten Spiegelflächen 44L, 44R jeweils genau zwei unterschiedliche Krümmungsradien m1L, m2L bzw. m1R, m2R aufweisen. Vielmehr können die gekrümmten Spiegelflächen 44L, 44R alternativ jeweils auch mehr als zwei unterschiedliche Krümmungsradien aufweisen. Auch können sich die Krümmungsradien der einen gekrümmten Spiegelfläche (hier z. B. 44L) in Anzahl und/oder Radius von den Krümmungsradien der anderen gekrümmten Spiegelfläche (hier z. B. 44R) unterscheiden. Weiter ist ein Unterschied zwischen den unterschiedlichen Krümmungsradien einer jeweiligen gekrümmten Spiegelfläche nicht auf das vorstehende Beispiel beschränkt. Allgemein ist es gemäß der vorliegenden Anmeldung ausreichend, wenn ein Unterschied zwischen wenigstens zwei unterschiedlichen, vorzugsweise zueinander im wesentlichen orthogonalen Krümmungsradien einer jeweiligen gekrümmten Spiegelfläche mindestens 5% und bevorzugt mindestens 10% und besonders bevorzugt mindestens 20% des größeren Krümmungsradius der wenigstens zwei Krümmungsradien beträgt. In diesem Zusammenhang bedeutet "im wesentlichen orthogonal", dass sich die beiden unterschiedlichen Krümmungsradien unter einem Winkel von zwischen 65° und 115° und bevorzugt von zwischen 80° und 100° und besonders bevorzugt von 90° schneiden.

Ersichtlich sind die Spiegelflächen 44L, 44R mit derartigen Krümmungsradien m1L, m2L bzw. m1R, m2R weder torisch noch sphärisch und weisen eine von Null verschiedene Brennweite auf. Kennzeichnend für die Spiegelflächen 44L, 44R der ersten und zweiten gekrümmten optischen Spiegel 40L, 40R ist, dass die gekrümmten Spiegelflächen 44L, 44R hinsichtlich des durch sie jeweils gefalteten Abbildungsstrahlengangs 14L, 14R jeweils eine rotationssymmetrische Wirkung aufweisen. Dabei ist die Krümmung der Spiegelflächen 44L, 44R jeweils so gewählt, dass eine Parallaxenfreiheit eines in dem jeweiligen linken und rechten Abbildungsstrahlengang 14L und 14R geführten und von der jeweiligen ersten und zweiten gekrümmten Spiegelfläche 44L, 44R reflektierten Abbildungsstrahlenbündels innerhalb eines Arbeitsbereichs des Systems 1' immer bestehen bleibt.

In der in Figur 4 gezeigten Ausführungsform sind die erste gekrümmte Spiegelfläche 44L und die zweite gekrümmte Spiegelfläche 44R identisch und bezüglich der Symmetrieebene 22 des stereoskopischen optischen Systems 1' symmetrisch angeordnet.

Dabei sind die ersten und zweiten gekrümmten Spiegelflächen 44L, 44R voneinander um einen Abstand A' beabstandet und relativ zueinander um einen Winkel β' geneigt. Durch den Abstand A' hindurch ist der Bestrahlungs-Strahlengang 27 für die von der Strahlenquelle 25 emittierte Strahlung geführt. Dabei können sowohl der Abstand A' als auch der Winkel β' sowohl konstant als auch variabel sein.

Gemäß dieser Ausführungsform sind die ersten und zweiten gekrümmten Spiegelflächen 44L, 44R weiter so angeordnet, dass eine (nicht gezeigte) Verbindungsgerade zwischen einem (nicht gezeigten) Flächenschwerpunkt der ersten gekrümmten Spiegelfläche 44L und einem (nicht gezeigten) Flächenschwerpunkt der zweiten gekrümmten Spiegelfläche 44R, und eine (nicht gezeigte) Verbindungsgerade zwischen einem (nicht gezeigten) minimalen Krümmungsmittelpunkt (Mittelpunkt des durch die gekrümmte Spiegelfläche am Flächenschwerpunkt der gekrümmten Spiegelfläche festgelegten kleinsten Krümmungskreises) der ersten gekrümmten Spiegelfläche 44L und einem (nicht gezeigten) minimalen Krümmungsmittelpunkt der zweiten gekrümmten Spiegelfläche 44R nicht co-axial, sondern um mehr als 0,1% eines minimalen Krümmungsradius am Flächenschwerpunkt der gekrümmten Spiegelflächen 44L, 44R beabstandet sind. Die vorliegende Erfindung ist jedoch nicht auf eine derartige Anordnung und Ausbildung der ersten und zweiten Spiegelfläche 44L, 44R beschränkt. Beispielsweise können die Verbindungsgerade der Flächenschwerpunkte und die Verbindungsgerade der minimalen Krümmungsmittelpunkte der Spiegelflächen parallel versetzt, gewinkelt oder windschief zueinander sein und/oder um mehr als 1% und insbesondere um mehr als 5% eines minimalen Krümmungsradius der gekrümmten Spiegelflächen am jeweiligen Flächenschwerpunkt beabstandet sein.

Der erste gekrümmte Spiegel 40L ist von der dritten off-axis Linse 5L um einen ersten Abstand D1' beabstandet. Entsprechend ist der zweite gekrümmte Spiegel 40R von der vierten off-axis Linse 5R um einen zweiten Abstand D2' beabstandet. Mittels des ersten Motors 21L können der erste gekrümmte Spiegel 40L und die dritte off-axis Linse 5L relativ zueinander verlagert werden, um den ersten Abstand D1' zu ändern. Entsprechend können mittels des zweiten Motors 21R der zweite gekrümmte Spiegel 40R und die vierte off-axis Linse 5R relativ zueinander verlagert werden, um den zweiten Abstand D2' zu ändern. Auch hier kann eine mechanischen oder elektrische Kopplung der ersten und zweiten Abstände D1' und D2' vorteilhaft sein oder kann alternativ auch ein gemeinsamer Motor verwendet werden (nicht gezeigt). Anstelle eines Motors kann in allen Ausführungsformen auch ein manueller Antrieb verwendet werden. Wie bei der vorstehend beschriebenen ersten Ausführungsform ermöglicht eine Veränderung der Abstände D1' und D2' eine Anpassung an einen jeweiligen Arbeitsabstand des stereoskopischen optischen Systems 1' von der Objektebene 23 und damit eine Fokussierung. Dabei stellt der erfindungsgemäße Aufbau bei geeigneter Wahl der für den ersten gekrümmten Spiegel 40L und die dritte off-axis Linse 5L bzw. für den zweiten gekrümmten Spiegel 40R und die vierte off-axis Linse 5R jeweils verwendeten optischen Oberflächen 44L, 18L bzw. 44R, 18R sicher, dass sich Hauptstrahlen des linken und rechten Abbildungsstrahlenganges 14L, 14R auch nach der Änderung des Arbeitsabstandes und damit nach einer Änderung der Abstände D1' und D2' (innerhalb eines Arbeitsbereichs des Systems 1') automatisch immer in der Objektebene 23 schneiden und dabei einen von Null verschiedenen Stereowinkel α einschließen. Aufgrund der Faltung des linken und rechten Abbildungsstrahlenganges 14L, 14R verlagert sich ein Schwerpunkt des stereoskopischen optischen Systems 1' bei einer Änderung der Abstände D1' und D2' nicht oder nur geringfügig.

Weiter sind in der ersten alternativen Ausführungsform die ersten und zweiten Prismen 6L, 6R jeweils durch ein Paar optischer Planspiegel 41L und 42L bzw. 41R und 42R ersetzt, die jeweils von einer gemeinsamen Fassung 43L bzw. 43R getragen werden und so einen konstanten Winkel δ1 bzw. δ2 von 90° einschließen. Dieser Winkel δ1, δ2 ist jedoch nicht auf genau 90° beschränkt. Vielmehr kann der Winkel δ1, δ2 alternativ ein beliebiger Winkel insbesondere zwischen 85° und 95° sein.

Die Verwendung erster und zweiter gekrümmter Spiegel 40L, 40R anstelle der ersten und zweiten off-axis Linsen 4L, 4R und anstelle der benachbarten optischen Umlenkelemente 3L, 3R führt zu einer deutlichen Gewichtseinsparung des stereoskopischen optischen Systems 1'. Dies ist insbesondere bei Verwendung des stereoskopischen optischen Systems 1' als Kopflupe von Vorteil.

Im Folgenden wird unter Bezugnahme auf die Figur 5 ein Strahlengang durch ein stereoskopisches optisches System 1" gemäß einer zweiten alternativen Ausführungsform beschrieben. Dabei wird auf eine Beschreibung von Elementen verzichtet, die denen der in Figur 4 gezeigten ersten alternativen Ausführungsform entsprechen.

Das stereoskopische optische System 1" gemäß der in Figur 5 gezeigten zweiten alternativen Ausführungsform unterscheidet sich von dem stereoskopischen optischen System 1' gemäß der vorstehend beschriebenen ersten alternativen Ausführungsform insbesondere dadurch, dass zusätzlich die dritte und vierte off-axis Linse 5L, 5R und die den off-axis Linsen 5L, 5R jeweils benachbarten optischen Planspiegel 41L, 41R in dem linken und rechten Abbildungsstrahlengang 14L und 14R jeweils paarweise durch dritte und vierte gekrümmte optische Spiegel 45L, 45R ersetzt sind. Die dritten und vierten gekrümmten Spiegel 45L, 45R weisen jeweils eine dritte und vierte gekrümmte optische Spiegelfläche 46L, 46R auf, welche jeweils so gewählt ist, dass eine Parallaxenfreiheit eines in dem jeweiligen linken und rechten Abbildungsstrahlengang 14L und 14R geführten und von der jeweiligen dritten und vierten gekrümmten Spiegelfläche 46L, 46R reflektierten Abbildungsstrahlenbündels innerhalb eines Arbeitsbereichs des Systems 1" immer bestehen bleibt. Somit weisen auch die dritten und vierten gekrümmten Spiegelflächen 46L, 46R der dritten und vierten gekrümmten optischen Spiegel 45L, 45R hinsichtlich des durch sie jeweils gefalteten Abbildungsstrahlengangs 14L, 14R jeweils eine rotationssymmetrische Wirkung auf. Hinsichtlich einer Auswahl von (in der Figur 5 nicht eigens dargestellten) Krümmungsradien für die dritte und vierte gekrümmte Spiegelfläche 46L, 46R wird auf die Ausführungen zu den Krümmungsradien m1L, m2L bzw. m1R, m2R der ersten und zweiten gekrümmten Spiegelflächen 44L, 44R in der Ausführungsform von Figur 4 verwiesen.

In der in Figur 5 gezeigten Ausführungsform sind die dritte gekrümmte Spiegelfläche 46L und die vierte gekrümmte Spiegelfläche 46R identisch und bezüglich der Symmetrieebene 22 des stereoskopischen optischen Systems 1" symmetrisch angeordnet. Der dritte gekrümmte Spiegel 45L und der Planspiegel 42L sowie der vierte gekrümmte Spiegel 45R und der Planspiegel 42R werden jeweils paarweise über eine gemeinsame Fassung 43L, 43R getragen.

Gemäß dieser Ausführungsform entspricht die Anordnung der dritten und vierten gekrümmten Spiegelflächen 46L und 46R relativ zueinander bezüglich der Verbindungsgerade der Flächenschwerpunkte und der Verbindungsgerade der minimalen Krümmungsmittelpunkte der Spiegelflächen 46L und 46R der vorstehend beschriebenen Anordnung der ersten und zweiten gekrümmten Spiegelfläche 44L, 44R relativ zueinander.

Der erste gekrümmte Spiegel 40L ist von dem dritten gekrümmten Spiegel 45L um einen variablen ersten Abstand D1" beabstandet. Entsprechend ist der zweite gekrümmte Spiegel 40R von dem vierten gekrümmten Spiegel 45R um einen variablen zweiten Abstand D2" beabstandet. Mittels eines ersten Motors 21L' können der dritte gekrümmte Spiegel 45L und der Planspiegel 42L relativ zu dem ersten gekrümmten Spiegel 40L verlagert werden, um den ersten Abstand D1" zu ändern. Entsprechend können der vierte gekrümmte Spiegel 45R und der Planspiegel 42R mittels eines zweiten Motors 21R' relativ zu dem zweiten gekrümmten Spiegel 40R verlagert werden, um den zweiten Abstand D2" zu ändern. Dabei kann in Folge der Verlagerung der Planspiegel 42L und 42R eine Vergrößerungsänderung des linken und rechten Abbildungsstrahlenganges 14L, 14R auftreten, welche durch das erste und zweite Zoomsystem 24L, 24R korrigiert werden kann. Auch hier kann eine mechanischen oder elektrische Kopplung der ersten und zweiten Abstände D1' und D2' vorteilhaft sein oder auch ein gemeinsamer Antrieb verwendet werden (nicht gezeigt). Wie bei den vorangegangenen Ausführungsformen ermöglicht eine Veränderung der Abstände D1" und D2" eine Anpassung des stereoskopischen optischen Systems 1" an einen jeweiligen Arbeitsabstand von der Objektebene 23 und damit eine Fokussierung. Dabei stellt der erfindungsgemäße Aufbau bei geeigneter Wahl der für den ersten gekrümmten Spiegel 40L und den dritten gekrümmten Spiegel 45L bzw. für den zweiten gekrümmten Spiegel 40R und den vierten gekrümmten Spiegel 45R jeweils verwendeten optischen Oberflächen 44L, 46L bzw. 44R, 46R sicher, daß Hauptstrahlen des linken und rechten Abbildungsstrahlenganges 14L, 14R auch nach der Änderung des Arbeitsabstandes und damit nach einer Änderung der Abstände D1' und D2' automatisch in der Objektebene 23 einen von Null verschiedenen Stereowinkel α einschließen.

Die Verwendung dritter und vierter gekrümmter Spiegel 45L, 45R anstelle der dritten und vierten off-axis Linsen 5L, 5R und benachbarten Planspiegel 41L, 41R führt gegenüber der ersten alternativen Ausführungsform zu einer weiteren Gewichtseinsparung des stereoskopischen optischen Systems 1". Dies ist insbesondere bei Verwendung des stereoskopischen optischen Systems 1" als Kopflupe von Vorteil.

Wie in Figur 2 gezeigt, kann das vorstehend beschriebene stereoskopische optische System 1, 1', 1" in ein Gehäuse 28 integriert sein, welches mittels eines Riemens am Kopf eines Benutzers derart befestigbar ist, daß ein linkes bzw. rechtes Auge 13L und 13R des Betrachters in jeweils das linke bzw. rechte Austrittsokular des stereoskopischen optischen Systems 1, 1', 1" blickt. Somit kann das stereoskopische optische System 1, 1', 1" Teil einer Kopflupe sein. Dabei hat die vorstehend beschriebene Faltung der linken und rechten Abbildungsstrahlengänge 14L und 14R zur Folge, daß die optischen Elemente des stereoskopischen optischen Systems 1 überwiegend quer zum Kopf des Benutzers und damit parallel zu einer durch eine Stirn des Benutzers aufgespannten Stirnebene angeordnet sind. Dies hat zur Folge, daß ein Gewicht der optischen Elemente nur mit einem kurzen Hebelarm auf den Kopf des Benutzers wirkt. Weiter verändert sich der Hebelarm auch bei einer Verlagerung der optischen Elemente nicht oder nur geringfügig. Eine vorzeitige Ermüdung des Benutzers und eine Verkrampfung der Nackenmuskulatur kann so vermieden werden.

Alternativ kann das vorstehend beschriebene stereoskopische optische System 1, 1', 1" auch in ein Operationsmikroskop und insbesondere ein digitales Operationsmikroskop integriert sein.

## Patentansprüche

1. Stereoskopisches optisches System (1; 1'; 1"), umfassend:
ein erstes optisches Teilsystem (2L) mit einer Mehrzahl von optischen Elementen (3L-12L; 40L, 41L, 42L, 40L) zur Bereitstellung eines linken Abbildungsstrahlengangs (14L) des stereoskopischen optischen Systems (1; 1'; 1"), wobei das erste optische Teilsystem (2L) zur Faltung des linken Abbildungsstrahlengangs (14L) wenigstens ein erstes Umlenkelement (3L, 10L; 40L, 10L) sowie eine erste Umlenkeinrichtung (6L; 41 L, 42L; 45L, 42L) aufweist; und
ein zweites optisches Teilsystem (2R) mit einer Mehrzahl von optischen Elementen (3R-12R; 40R, 41R, 42R, 45R) zur Bereitstellung eines rechten Abbildungsstrahlengangs (14R) des stereoskopischen optischen Systems (1), wobei das zweite optische Teilsystem (2R) zur Faltung des rechten Abbildungsstrahlengangs (14R) wenigstens ein zweites Umlenkelement (3R, 10R; 40R, 10R) sowie eine zweite Umlenkeinrichtung (6R; 41 R, 42R; 45R, 42R) aufweist,;
wobei die optischen Elemente (3L-12L; 40L, 5L, 41 L, 42L, 7L-12L; 40L, 45L) des ersten optischen Teilsystems (2L) und die optischen Elemente (3R-12R; 40R, 5R, 41R, 42R, 7R-12R; 40R, 45R) des zweiten optischen Teilsystems (2R) bezüglich einer gemeinsamen Symmetrieebene (22) im wesentlichen symmetrisch angeordnet sind;
**dadurch gekennzeichnet,**
**dass** ein Hauptstrahl des linken Abbildungsstrahlengangs (14L) zwischen dem wenigstens einen ersten Umlenkelement (3L, 10L; 40L, 10L) sowie der ersten Umlenkeinrichtung (6L; 41 L, 42L; 45L, 42L) und ein Hauptstrahl des rechten Abbildungsstrahlengangs (14R) zwischen dem wenigstens einen zweiten Umlenkelement (3R, 10R; 40R, 10R) sowie der zweiten Umlenkeinrichtung (6R; 41 R, 42R; 45R, 42R) mit der gemeinsamen Symmetrieebene (22) jeweils einen kleinsten Winkel (γ1, γ2, η1, η2) von zwischen 45° und 135° und bevorzugt zwischen 75° und 105° und besonders bevorzugt 90° einschließen und/oder jeweils in Ebenen liegen, die mit der gemeinsamen Symmetrieebene (22) jeweils einen kleinsten Winkel (γ1, γ2, η1, η2) von zwischen 45° und 135° und bevorzugt zwischen 75° und 105° und besonders bevorzugt 90° einschließen; und
**dass** zwischen dem wenigstens einen ersten Umlenkelement (3L, 10L; 40L, 10L) und der ersten Umlenkeinrichtung (6L; 41L, 42L; 45L, 42L) und dem wenigstens einen zweiten Umlenkelement (3R, 10R; 40R, 10R) und der zweiten Umlenkeinrichtung (6R; 41 R, 42R; 45R, 42R) jeweils wenigstens eine optische Anordnung variabler Brechkraft (4L, 5L, 7L-9L, 4R, 5R, 7R-9R) angeordnet ist;
wobei die optische Anordnung variabler Brechkraft (4L, 5L, 7L-9L, 4R, 5R, 7R-9R) jeweils wenigstens zwei entlang des linken und rechten Abbildungsstrahlenganges relativ zueinander verlagerbare Linsen (4L, 5L, 7L-9L, 4R, 5R, 7R-9R) umfasst.

2. Stereoskopisches optisches System (1,1'; 1") nach Anspruch 1,
wobei das erste optische Teilsystem (2L) wenigstens ein erstes und ein drittes Umlenkelement (3L, 10L; 40L, 10L) aufweist, und die erste Umlenkeinrichtung (6L; 41L, 42L; 45L, 42L) entlang des linken Abbildungsstrahlengangs (14L) zwischen den beiden ersten und dritten Umlenkelementen (3L, 10L; 40L, 10L) angeordnet ist;
wobei das zweite optische Teilsystem (2R) wenigstens ein zweites und ein viertes Umlenkelement (3R, 10R; 40R, 10R) aufweist, und die zweite Umlenkeinrichtung (6R; 41 R, 42R; 45R, 42R) entlang des rechten Abbildungsstrahlengangs (14R) zwischen den beiden zweiten und vierten Umlenkelementen (3R, 10R; 40R, 10R) angeordnet ist; und
wobei jeweils Hauptstrahlen des linken Abbildungsstrahlengangs (14L) zwischen dem ersten bzw. dritten Umlenkelement (3L, 10L; 40L, 10L) und der ersten Umlenkeinrichtung (6L; 41 L, 42L; 45L, 42L) sowie jeweils Hauptstrahlen des rechten Abbildungsstrahlengangs (14R) zwischen dem zweiten bzw. vierten Umlenkelement (3R, 10R; 40R, 10R) und der zweiten Umlenkeinrichtung (6R; 41 R, 42R; 45R, 42R) mit der gemeinsamen Symmetrieebene (22) jeweils einen kleinsten Winkel (γ1, γ2, η1, η2) von zwischen 45° und 135° und bevorzugt zwischen 75° und 105° und besonders bevorzugt 90° einschließen und/oder jeweils in Ebenen liegen, die mit der gemeinsamen Symmetrieebene (22) jeweils einen kleinsten Winkel (γ1, γ2, η1, η2) von zwischen 45° und 135° und bevorzugt zwischen 75° und 105° und besonders bevorzugt 90° einschließen.

3. Stereoskopisches optisches System (1. 1'; 1") nach einem der Ansprüche 1 oder 2,
wobei die wenigstens eine optische Anordnung variabler Brechkraft (4L, 5L, 7L-9L, 4R, 5R, 7R-9R) jeweils eine verlagerbare optische Linse (7L, 8L, 9L, 7R, 8R, 9R) eines ersten bzw. zweiten Zoomsystems (24L, 24R) ist, welches jeweils im linken bzw. rechten Abbildungsstrahlengang (14L, 14R) zwischen dem wenigstens jeweils einen Umlenkelement (10L, 10R) und der ersten bzw. zweiten Umlenkeinrichtung (6L, 6R; 41 L, 42L, 41 R, 42R; 45L, 42L, 45R, 42L) angeordnet ist, und jeweils relativ zueinander verlagerbare optische Zoom-Elemente (7L-9L, 7R-9R) aufweist, um eine variable Abbildungsvergrößerung des linken bzw. rechten Abbildungsstrahlengangs (14L, 14R) zu bewirken, und
wobei die ersten optischen Zoom-Elemente (7L, 8L, 9L) des ersten Zoomsystems (24L) im linken Abbildungsstrahlengang (14L) entlang einer gemeinsamen optischen Achse angeordnet sind, die mit der gemeinsamen Symmetrieebene (22) einen kleinsten Winkel (γ1) von zwischen 45° und 135° und bevorzugt zwischen 75° und 105° und besonders bevorzugt 90° einschließt und/oder die in einer Ebene liegt, welche mit der gemeinsamen Symmetrieebene (22) einen kleinsten Winkel (γ1) von zwischen 45° und 135° und bevorzugt zwischen 75° und 105° und besonders bevorzugt 90° einschließt; und wobei die zweiten optischen Zoom-Elemente (7R, 8R, 9R) des zweiten Zoomsystems (24R) im rechten Abbildungsstrahlengang (14R) entlang einer gemeinsamen optischen Achse angeordnet sind, die mit der gemeinsamen Symmetrieebene (22) einen kleinsten Winkel (γ2) von zwischen 45° und 135° und bevorzugt zwischen 75° und 105° und besonders bevorzugt 90° einschließt und/oder die in einer Ebene liegt, welche mit der gemeinsamen Symmetrieebene (22) einen kleinsten Winkel (γ2) von zwischen 45° und 135° und bevorzugt zwischen 75° und 105° und besonders bevorzugt 90° einschließt.

4. Stereoskopisches optisches System (1.1'; 1 ") nach Anspruch 3,
wobei die ersten optischen Zoom-Elemente (7L, 8L, 9L) des ersten Zoomsystems (24L) zwischen dem dritten Umlenkelement (10L) und der ersten Umlenkeinrichtung (6L; 41 L, 42L; 45L, 42L) entlang einer gemeinsamen optischen Achse angeordnet sind, die zu einem in dem linken Abbildungsstrahlengang (14L) zwischen dem ersten Umlenkelement (3L; 40L) und der ersten Umlenkeinrichtung (6L; 41 L, 42L; 45L, 42L) geführten Hauptstrahl parallel versetzt ist; und
wobei die zweiten optischen Zoom-Elemente (7R, 8R, 9R) des zweiten Zoomsystems (24R) zwischen dem vierten Umlenkelement (10R) und der zweiten Umlenkeinrichtung (6R; 41 R, 42R; 45R, 42R) entlang einer gemeinsamen optischen Achse angeordnet sind, die zu einem in dem rechten Abbildungsstrahlengang (14R) zwischen dem zweiten Umlenkelement (3R; 40R) und der zweiten Umlenkeinrichtung (6R; 41 R, 42R; 45R, 42R) geführten Hauptstrahl parallel versetzt ist.

5. Stereoskopisches optisches System (1, 1') nach einem der Ansprüche 1 oder 2, wobei die wenigstens eine optische Anordnung variabler Brechkraft (4L, 5L, 4R, 5R; 5L, 5R) jeweils eine zur Einstellung eines Arbeitsabstandes des stereoskopischen optischen Systems (1; 1') relativ zu einem weiteren optischen Element (5L, 4L, 5R, 4R; 40L, 40R) verlagerbare optische Linse (4L, 5L, 4R, 5R; 5L, 5R) ist.

6. Stereoskopisches optisches System (1, 1') nach einem der Ansprüche 1 oder 2, wobei die wenigstens eine optische Anordnung variabler Brechkraft jeweils eine Flüssigkristalllinse und/oder eine Flüssigkeitslinse ist, deren Brechkraft durch Ansteuern einstellbar ist.

7. Stereoskopisches optisches System (1,1'; 1") nach einem der Ansprüche 1 bis 6, wobei die ersten und zweiten Umlenkeinrichtungen (6L, 6R; 41L, 42L, 41R, 42R;
45L, 42L, 45R, 42R) jeweils ausgebildet sind, um eine Faltung des linken bzw. rechten Abbildungsstrahlengangs (14L, 14R) um zwischen 135° und 225° und bevorzugt zwischen 170° und 190° und besonders bevorzugt 180° zu bewirken; und
die ersten und zweiten und/oder die dritten und vierten Umlenkelemente (3L, 10L, 3R, 10R; 40L, 10L, 40R, 10R) jeweils ausgebildet sind, um eine Faltung des linken bzw. rechten Abbildungsstrahlengangs (14L, 14R) um zwischen 65° und 115° und bevorzugt zwischen 80° und 100° und besonders bevorzugt 90° zu bewirken.

8. Stereoskopisches optisches System (1,1'; 1 ") nach Anspruch 7, wobei die ersten und zweiten Umlenkeinrichtungen (6L, 6R; 42L, 45L, 42R, 45R) jeweils zwei optische Spiegelflächen aufweisen, die miteinander jeweils einen Winkel (δ1, δ2) zwischen 85° und 95° und bevorzugt 90° einschließen.

9. Stereoskopisches optisches System (1,1'; 1") nach einem der Ansprüche 1 bis 8, wobei die erste und zweite Umlenkeinrichtung (6L, 6R; 41 L, 42L, 41 R, 42R; 45L, 42L, 45R, 42R) und/oder eine erste und zweite Spiegelfläche des ersten und zweiten Umlenkelements (3L, 3R; 40L, 40R) bezogen auf eine gemeinsame Symmetrieebene (22) symmetrisch angeordnet sind.

10. Stereoskopisches optisches System (1,1'; 1") nach einem der Ansprüche 1 bis 9, wobei ein Winkel (β) zwischen einer ersten Spiegelfläche des ersten Umlenkelements (3L; 40L) und einer zweiten Spiegelfläche des zweiten Umlenkelements (3R; 40R) zwischen 50° und 130° und insbesondere zwischen 80° und 100° und bevorzugt 90° beträgt.

11. Stereoskopisches optisches System (1; 1'; 1") nach einem der Ansprüche 1 bis 10, wobei das System (1; 1'; 1") ein Bestrahlungssystem mit einer Strahlenquelle (25) zum Emittieren von Strahlung und mit einer Bestrahlungs-Optik (26) zum Bereitstellen eines Bestrahlungs-Strahlenganges (27) für die von der Strahlenquelle (25) emittierte Strahlung aufweist.

12. Stereoskopisches optisches System (1; 1'; 1") nach Anspruch 11,
wobei erste und zweite Spiegelflächen des ersten und zweiten Umlenkelements (3L, 3R; 40L, 40R) jeweils zumindest bereichsweise für von dem Bestrahlungs-Strahlengang (27) geführte Strahlung eine höhere Transparenz aufweisen, als für im linken bzw. rechten Abbildungsstrahlengang (14L, 14R) geführte Strahlung und/oder voneinander um einen Abstand (A) größer Null beabstandet sind; und
wobei der von der Bestrahlungs-Optik (26) bereitgestellte Bestrahlungs-Strahlengang (27) durch die erhöhte Transparenz der ersten und zweiten Spiegelflächen hindurch und/oder durch den Abstand (A) zwischen den beiden ersten und zweiten Spiegelflächen hindurch verläuft.

13. Stereoskopisches optisches System (1'; 1") nach einem der Ansprüche 1 bis 12, wobei wenigstens ein Umlenkelement (3L, 3R, 10L, 10R; 40L, 40R, 10L, 10R) und/oder wenigstens eine Umlenkeinrichtung (6L, 6R; 41 L, 41 R, 42L, 42R; 45L, 45R, 42L, 42R) des ersten und zweiten optischen Teilsystems (2L, 2R) jeweils eine gekrümmte Spiegelfläche (44L, 44R, 46L, 46R) mit einem Krümmungsradius (m1 L, m2L, m1R, m2R) kleiner als zehn Meter und vorzugsweise kleiner als ein Meter aufweist, und welche gekrümmte Spiegelfläche (44L, 44R, 46L, 46R) hinsichtlich des durch sie jeweils gefalteten Abbildungsstrahlengangs (14L, 14R) eine rotationssymmetrische Wirkung aufweist.

14. Stereoskopisches optisches System (1'; 1 ") nach einem der Ansprüche 1 bis 13,
wobei das erste optische Teilsystem (2L) weiter ein erstes Okularsystem (11 L, 12L) und das zweite optische Teilsystem (2R) weiter ein zweites Okularsystem (11 R, 12R) aufweist; und
wobei ein Hauptstrahl des in dem ersten Okularsystem (11L, 12L) geführten linken Abbildungsstrahlengangs (14L) sowie ein Hauptstrahl des in dem zweiten Okularsystem (11R, 12R) geführten rechten Abbildungsstrahlengangs (14R) im wesentlichen parallel zueinander verlaufen.

15. Stereoskopisches optisches System (1; 1'; 1") nach einem der Ansprüche 1 bis 14, wobei Hauptstrahlen der linken und rechten Abbildungsstrahlengänge (14L, 14R) im wesentlichen in einer gemeinsamen Ebene liegen.

## Claims

1. A stereoscopic optical system (1; 1'; 1") comprising:
a first optical subsystem (2L) having a plurality of optical elements (3L-12L; 40L, 41 L, 42L; 40L,) for providing a left imaging optical path (14L) of the stereoscopic optical system (1; 1'; 1"), wherein the first optical subsystem (2L) comprises at least a first deflecting element (3L, 10L; 40L, 10L) as well as a first deflecting device (6L; 41 L, 42L; 45L, 42L) for folding the left imaging optical path (14L); and
a second optical subsystem (2R) having a plurality of optical elements (3R-12R; 40R, 41 R, 42R; 45R) for providing a right imaging optical path (14R) of the stereoscopic optical system (1), wherein the second optical subsystem (2R) comprises at least a second deflecting element (3R, 10R; 40R, 10R) as well as a second deflecting device (6R; 41 R, 42R; 45R, 42R) for folding the right imaging optical path (14R);
wherein the optical elements (3L-12L; 40L, 5L, 41 L, 42L, 7L-12L; 40L, 45L) of the first optical subsystem (2L) and the optical elements (3R-12R; 40R, 5R, 41 R, 42R, 7R-12R; 40R, 45R) of the second optical subsystem (2R) are arranged substantially symmetrically with respect to a common plane of symmetry (22);
**characterized in that**
a main beam of the left imaging optical path (14L) between the at least one first deflecting element (3L, 10L; 40L, 10L) and the first deflecting device (6L; 41 L, 42L; 45L, 42L) and a main beam of the right imaging optical path 14R) between the at least one second deflecting element (3R, 10R; 40R, 10R) and the second deflecting device (6R; 41 R, 42R; 45R, 42R) respectively include a smallest angle (γ1, γ2, η1, η2) between 45° and 135°, preferably between 75° and 105°, and particularly preferred 90°, with the common plane of symmetry (22) and/or respectively lie in planes which include a smallest angle (γ1, γ2, η1, η2) between 45° and 135°, preferably between 75° and 105°, and particularly preferred 90°, with the common plane of symmetry (22); and
**in that** between the at least one first deflecting element (3L, 10L; 40L, 10L) and the first deflecting device (6L; 41 L, 42L; 45L, 42L) and the at least one second deflecting element (3R, 10R; 40R, 10R) and the second deflecting device (6R; 41 R, 42R; 45R, 42R) at least one optical arrangement of variable refraction (4L, 5L, 7L-9L, 4R, 5R, 7R-9R) is respectively disposed;
wherein the arrangement of variable refraction (4L, 5L, 7L-9L, 4R, 5R, 7R-9R) comprises at least two lenses (4L, 5L, 7L-9L, 4R, 5R, 7R-9R) which are displaceable along the left and right imaging optical paths respectively.

2. The stereoscopic optical system (1, 1'; 1") according to claim 1,
wherein the first optical subsystem (2L) comprises at least a first and a third deflecting element (3L, 10L; 40L, 10L), and the first deflecting device (6L; 41 L, 42L; 45L, 42L) is disposed along the left imagining optical path (14L) between the two first and third deflecting elements (3L, 10L; 40L, 10L);
wherein the second optical subsystem (2R) comprises at least a second and a fourth deflecting element (3R, 10R; 40R, 10R), and the second deflecting device (6R; 41 R, 42R; 45R, 42R) is disposed along the right imaging optical path (14R) between the two second and fourth deflecting elements (3R, 10R; 40R, 10R); and
wherein main beams of the left imaging optical path (14L) between the first and the third deflecting element (3L, 10L; 40L, 10L) and the first defecting device (6L; 41 L, 42L; 45L, 42L) and main beams of the right imaging optical path (14R) between the second and the fourth deflecting elements (3R, 10R; 40R, 10R) and the second deflecting device (6R, 41 R, 42R; 45R, 42R) respectively include a smallest angle (γ1, γ2, η1, η2) between 45° and 135°, preferably between 75° and 105°, and particularly preferred 90°, with the common plane of symmetry (22) and/or respectively lie in planes which include a smallest angle (γ1, γ2, η1, η2) between 45° and 135°, preferably between 75° and 105°, and particularly preferred 90°, with the common plane of symmetry (22).

3. The stereoscopic optical system (1, 1'; 1") according to one of claims 1 or 2,
wherein the at least one optical arrangement of variable refraction (4L, 5L, 7L-9L, 4R, 5R, 7R-9R) is a displaceable optical lens (7L, 8L, 9L, 7R, 8R, 9R) of a first and a second zoom system (24L, 24R), respectively, which is disposed respectively in the left and the right imaging optical paths (14L, 14R) between the at least one deflecting element (10L, 10R) and the first and the second deflecting devices (6L, 6R; 41 L, 42L, 41 R, 42R; 45L, 42L, 45R, 42L) and comprises optical zoom elements (7L-9L, 7R-9R) which are displaceable relative to one another in order to cause a variable image magnification of the left and the right imaging optical paths (14L, 14R), respectively, and
wherein the first optical zoom element (7L, 8L, 9L) of the first zoom system (24L) is disposed in the left imaging optical path (14L) along a common optical axis which includes a smallest angle (γ1) between 45° and 135°, preferably between 75° and 105°, and particularly preferred 90°, with the common plane of symmetry (22); and/or lies in a plane which includes a smallest angle (γ1) between 45° and 135°, preferably between 75° and 105°, and particularly preferred 90°, with the common plane of symmetry (22); and
wherein the second optical zoom element (7R, 8R, 9R) of the second zoom system (24R) is disposed in the right imaging optical path (14R) along a common optical axis which includes a smallest angle (γ2) between 45° and 135°,
preferably between 75° and 105°, and particularly preferred 90°, with the common plane of symmetry (22) and/or lies in a plane which includes a smallest angle (γ2) between 45° and 135°, preferably between 75° and 105°, and particularly preferred 90°, with the common plane of symmetry (22).

4. The stereoscopic optical system (1, 1'; 1") according to claim 3,
wherein the first optical zoom elements (7L, 8L, 9L) of the first zoom system (24L) between the third deflecting element (10L) and the first deflecting device (6L; 41 L, 42L; 45L, 42L) are disposed along a common optical axis which is offset in parallel to a main beam extending in the left imaging optical path (14L) between the first deflecting element (3L; 40L) and the first deflecting device (6L; 41 L, 42L; 45L, 42L); and
wherein the second optical zoom elements (7R, 8R, 9R) of the second zoom system (24R) between the fourth deflecting element (10R) and the second deflecting device (6R; 41 R, 42R; 45R, 42R) are disposed along a common optical axis which is offset in parallel to a main beam extending in the right imaging optical path (14R) between the second deflecting element (3R; 40R) and the second deflecting device (6R; 41 R, 42R; 45R, 42R).

5. The stereoscopic optical system (1, 1') according to one of claims 1 or 2, wherein the at least one optical arrangement of variable refraction (4L, 5L, 4R, 5R; 5L, 5R) each is a displaceable optical lens (4L, 5L, 4R, 5R; 5L, 5R) that is displaceable relative to a further optical element (5L, 4L, 5R, 4R; 40L, 40R) for adjusting a working distance of the stereoscopic optical system (1;1').

6. The stereoscopic optical system (1, 1') according to one of claims 1 or 2, wherein the at least one optical arrangement of variable refraction each is a liquid crystal lens and/or liquid lens, the refraction power of which is adjustable by driving.

7. The stereoscopic optical system (1, 1'; 1") according to one of claims 1 to 6,
wherein the first and second deflecting devices (6L, 6R; 41 L, 42L, 41 R, 42R; 45L, 42L, 45R, 42R) each are configured such that they cause a folding of the left and right imaging optical paths (14L, 14R), respectively, by between 135° and 225° and preferably between 170° and 190° and particularly preferred 180°; and
the first and second and/or the third and fourth deflecting elements (3L, 10L, 3R, 10R; 40L, 10L, 40R, 10R) each are configured such that they cause a folding of the left and right imaging optical paths (14L, 14R), respectively, by between 65° and 115° and preferably between 80° and 100° and particularly preferred 90°.

8. The stereoscopic optical system (1, 1'; 1") according to claim 7, wherein the first and second deflecting devices (6L, 6R; 42L, 45L, 42R, 45R) each comprise two optical mirror surfaces, which respectively include an angle (δ1, δ2) between 85° and 95° and preferably 90° with one another.

9. The stereoscopic optical system (1, 1'; 1") according to one of claims 1 to 8, wherein the first and second deflecting devices (6L, 6R; 41 L, 42L, 41 R, 42R; 45L, 42L, 45R, 42R) and/or a first and second mirror surface of the first and second deflecting element (3L, 3R; 40L, 40R) are arranged symmetrically with respect to one common plane of symmetry (22).

10. The stereoscopic optical system (1, 1'; 1") according to one of claims 1 to 9, wherein an angle (β) between a first mirror surface of the first deflecting element (3L; 40L) and a second mirror surface of the second deflecting element (3R; 40R) amounts to between 50° and 130° and preferably between 80° and 100° and particularly preferred 90°.

11. The stereoscopic optical system (1; 1'; 1") according to one of claims 1 to 10, wherein the system (1; 1'; 1")comprises an irradiation system with an irradiation source (25) for emission of radiation and an irradiation optics (26) for providing an irradiation optical path (27) for the radiation emitted by the irradiation source (25).

12. The stereoscopic optical system (1; 1'; 1 ") according to claim 11,
wherein first and second mirror surfaces of the first and second deflecting elements (3L, 3R; 40L, 40R) at least in regions each have a higher transparency for radiation guided by the irradiation optical path (27) than for radiation guided in the left and right imaging optical path (14L, 14R) and/or are separated from one another by a distance (A) larger than zero; and
wherein the irradiation optical path (27) provided by the irradiation optics (26) is guided through the higher transparency of the first and second mirror surfaces and/or through the distance (A) between the two first and second mirror surfaces.

13. The stereoscopic optical system (1'; 1") according to one of claims 1 to 12, wherein at least one deflecting element (3L, 3R, 10L, 10R; 40L, 40R, 10L, 10R) and/or at least one deflecting device (6L, 6R; 41 L, 41 R, 42L, 42R; 45L, 45R, 42L, 42R) of the first and second optical subsystem (2L, 2R) each comprises a curved mirror surface (44L, 44R, 46L, 46R) having a radius of curvature (m1 L, m2L, m1 R, m2R) that is smaller than ten meters and preferably smaller than one meter, and which curved mirror surface (44L, 44R, 46L, 46R) has a rotational symmetric effect with respect to the imaging optical paths (14L, 14R) they respectively fold.

14. The stereoscopic optical system (1'; 1 ") according to one of claims 1 to 13,
wherein the first optical subsystem (2L) further comprises a first ocular system (11 L, 12L) and the second optical subsystem (2R) further comprises a second ocular system (11R, 12R); and
wherein a main beam of the left imaging optical path (14L) guided in the first ocular system (11L, 12L) as well as a main beam of the right imaging optical path (14R) guided in the second ocular system (11 R, 12R) are oriented substantially parallel to one another.

15. The stereoscopic optical system (1; 1'; 1") according to one of claims 1 to 14, wherein main beams of the left and right imaging optical paths (14L, 14R) are substantially located in one common plane.

## Revendications

1. Système optique stéréoscopique (1; 1'; 1"), comprenant:
- un premier sous-système optique (2L) avec une pluralité d'éléments optiques (3L à 12L; 40L, 41 L, 42L; 40L) pour fournir un chemin optique de reproduction gauche (14L) du système optique stéréoscopique (1; 1'; 1"), dans lequel le premier sous-système optique (2L) présente pour la convolution du chemin optique de reproduction gauche (14L) au moins un premier élément déflecteur (3L, 10L; 40L, 10L) ainsi qu'un premier dispositif déflecteur (6L; 41 L, 42L; 45L, 42L); et
- un deuxième sous-système optique (2R) avec une pluralité d'éléments optiques (3R à 12R; 40R, 41 R, 42R, 45R) pour fournir un chemin optique de reproduction droit (14R) du système optique stéréoscopique (1), dans lequel le deuxième sous-système optique (2R) présente pour la convolution du chemin optique de reproduction droit (14R) au moins un deuxième élément déflecteur (3R, 10R; 40R, 10R) ainsi qu'un deuxième dispositif déflecteur (6R; 41 R, 42R; 45R, 42R); dans lequel les éléments optiques (3L à 12L; 40L, 5L, 41 L, 42L, 7L à 12L; 40L, 45L) du premier sous-système optique (2L) et les éléments optiques (3R à 12R; 40R, 5R, 41 R, 42R, 7R à 12R; 40R, 45R) du deuxième sous-système optique (2R) sont disposés de manière substantiellement symétrique par rapport à un plan de symétrie commun (22);
**caractérisé en ce qu'**un rayon principal du chemin optique de reproduction gauche (14L) entre ledit au moins un premier élément déflecteur (3L, 10L; 40L, 10L) ainsi que ledit premier dispositif déflecteur (6L; 41 L, 42L; 45L, 42L) et un rayon principal du chemin optique de reproduction droit (14R) entre ledit au moins un deuxième élément déflecteur (3R, 10R; 40R, 10R) ainsi que ledit deuxième dispositif déflecteur (6R; 41 R, 42R; 45R, 42R) forment avec le plan de symétrie commun (22) respectivement un plus petit angle (γ1, γ2, η1, η2) d'entre 45° et 135° et de préférence d'entre 75° et 105° et de plus grande préférence de 90°, et/ou sont situés respectivement dans des plans formant avec le plan de symétrie commun (22) respectivement un plus petit angle (γ1, γ2, η1, η2) d'entre 45° et 135° et de préférence d'entre 75° et 105° et de plus grande préférence de 90°; et
**en ce qu'**entre ledit au moins un premier élément déflecteur (3L, 10L; 40L, 10L) et ledit premier dispositif déflecteur (6L; 41 L, 42L; 45L, 42L) et ledit au moins un deuxième élément déflecteur (3R; 10R, 40R; 10R) et ledit deuxième dispositif déflecteur (6R; 41 R, 42R; 45R, 42R) est disposé respectivement au moins un ensemble optique de puissance variable (4L, 5L, 7L à 9L, 4R, 5R, 7R à 9R);
dans lequel l'ensemble optique de puissance variable (4L, 5L, 7L à 9L, 4R, 5R, 7R à 9R) comprend respectivement au moins deux lentilles (4L, 5L, 7L à 9L, 4R, 5R, 7R à 9R) déplaçables l'une par rapport à l'autre suivant les chemins optiques de reproduction gauche et droit.

2. Système optique stéréoscopique (1, 1'; 1") selon la revendication 1,
dans lequel le premier sous-système optique (2L) présente au moins un premier et un troisième élément déflecteur (3L, 10L; 40L, 10L), et le premier dispositif déflecteur (6L; 41 L, 42L; 45L, 42L) est disposé suivant le chemin optique de reproduction gauche (14L) entre les deux premier et troisième éléments déflecteurs (3L, 10L; 40L, 10L);
dans lequel le deuxième sous-système optique (2R) présente au moins un deuxième et un quatrième élément déflecteur (3R, 10R; 40R, 10R), et le deuxième dispositif déflecteur (6R; 41 R, 42R; 45R, 42R) est disposé suivant le chemin optique de reproduction droit (14R) entre les deux deuxième et quatrième éléments déflecteurs (3R, 10R; 40R, 10R); et
dans lequel respectivement les rayons principaux du chemin optique de reproduction gauche (14L) entre le premier ou troisième élément déflecteur (3L, 10L; 40L, 10L) et le premier dispositif déflecteur (6L; 41 L, 42L; 45L, 42L), ainsi que respectivement les rayons principaux du chemin optique de reproduction droit (14R) entre le deuxième ou quatrième élément déflecteur (3R, 10R; 40R, 10R), et le deuxième dispositif déflecteur (6R; 41 R, 42R; 45R, 42R) forment avec le plan de symétrie commun (22) respectivement un plus petit angle (γ1, γ2, η1, η2) d'entre 45° et 135° et de préférence d'entre 75° et 105° et de plus grande préférence de 90°, et/ou sont situés respectivement dans des plans formant avec le plan de symétrie commun (22) respectivement un plus petit angle (γ1, γ2, η1, η2) d'entre 45° et 135° et de préférence d'entre 75° et 105° et de plus grande préférence de 90°.

3. Système optique stéréoscopique (1, 1'; 1") selon l'une quelconque des revendications 1 ou 2,
dans lequel ledit au moins un ensemble optique de puissance variable (4L, 5L, 7L à 9L, 4R, 5R, 7R à 9R) est respectivement une lentille optique déplaçable (7L, 8L, 9L, 7R, 8R, 9R) d'un premier ou deuxième système de zoom (24L, 24R) qui est agencé respectivement sur le chemin optique de reproduction gauche ou droit (14L, 14R) entre ledit au moins un élément déflecteur (10L, 10R) et ledit premier ou deuxième dispositif déflecteur (6L, 6R; 41 L, 42L, 41 R, 42R; 45L, 42L, 45R, 42L), et présente respectivement des éléments de zoom optiques (7L à 9L, 7R à 9R) respectivement déplaçables l'un par rapport à l'autre afin de provoquer un agrandissement de reproduction variable du chemin optique de reproduction gauche ou droit (14L, 14R), et
dans lequel les premiers éléments de zoom optiques (7L, 8L, 9L) du premier système de zoom (24L) sont disposés sur le chemin optique de reproduction gauche (14L) suivant un axe optique commun qui forme avec le plan de symétrie commun (22) un plus petit angle (γ1) d'entre 45° et 135° et de préférence d'entre 75° et 105° et de plus grande préférence de 90°, et/ou est situé dans un plan formant avec le plan de symétrie commun (22) un plus petit angle (γ1) d'entre 45° et 135° et de préférence d'entre 75° et 105° et de plus grande préférence de 90°; et
dans lequel les deuxièmes éléments de zoom optiques (7R, 8R, 9R) du deuxième système de zoom (24R) sont disposés sur le chemin optique de reproduction droit (14R) suivant un axe optique commun qui forme avec le plan de symétrie commun (22) un plus petit angle (γ2) d'entre 45° et 135° et de préférence d'entre 75° et 105° et de plus grande préférence de 90°, et/ou est situé dans un plan formant avec le plan de symétrie commun (22) un plus petit angle (γ2) d'entre 45° et 135° et de préférence d'entre 75° et 105° et de plus grande préférence de 90°.

4. Système optique stéréoscopique (1, 1'; 1") selon la revendication 3,
dans lequel les premiers éléments de zoom optiques (7L, 8L, 9L) du premier système de zoom (24L) sont disposés entre le troisième élément déflecteur (10L) et le premier dispositif déflecteur (6L; 41 L, 42L; 45L, 42L) suivant un axe optique commun qui est décalé en parallèle à un rayon principal dirigé sur le chemin optique de reproduction gauche entre le premier élément déflecteur (3L; 40L) et le premier dispositif déflecteur (6L; 41 L, 42L; 45L, 42L); et
dans lequel les deuxièmes éléments de zoom optiques (7R, 8R, 9R) du deuxième système de zoom (24R) sont disposés entre le quatrième élément déflecteur (10R) et le deuxième dispositif déflecteur (6R; 41 R, 42R; 45R, 42R) suivant un axe optique commun qui est décalé en parallèle à un rayon principal dirigé sur le chemin optique de reproduction droit entre le deuxième élément déflecteur (3R; 40R) et le deuxième dispositif déflecteur (6R; 41 R, 42R; 45R, 42R).

5. Système optique stéréoscopique (1, 1') selon l'une quelconque des revendications 1 ou 2, dans lequel ledit au moins un ensemble optique de puissance variable (4L, 5L, 4R, 5R; 5L, 5R) est respectivement une lentille optique (4L, 5L, 4R, 5R; 5L, 5R) déplaçable par rapport à un autre élément optique (5L, 4L, 5R, 4R; 40L, 40R) pour le réglage d'une distance de travail du système optique stéréoscopique (1; 1').

6. Système optique stéréoscopique (1, 1') selon l'une quelconque des revendications 1 ou 2, dans lequel ledit au moins un ensemble optique de puissance variable est respectivement une lentille à cristaux liquides et/ou une lentille liquide dont la puissance est réglable par excitation.

7. Système optique stéréoscopique (1, 1'; 1") selon l'une quelconque des revendications 1 à 6,
dans lequel les premier et deuxième dispositifs déflecteurs (6L, 6R; 41 L, 42L, 41 R, 42R; 45L, 42L, 45R, 42R) sont respectivement réalisés pour provoquer une convolution du chemin optique de reproduction gauche ou droit (14L, 14R) d'entre 135° et 225° et de préférence d'entre 170° et 190° et de plus grande préférence de 180°; et
les premier et deuxième et/ou les troisième et quatrième éléments déflecteurs (3L, 10L, 3R, 10R; 40L, 10L, 40R, 10R) sont respectivement réalisés pour provoquer une convolution du chemin optique de reproduction gauche ou droit (14L, 14R) d'entre 65° et 115° et de préférence d'entre 80° et 100° et de plus grande préférence de 90°.

8. Système optique stéréoscopique (1, 1'; 1") selon la revendication 7, dans lequel les premier et deuxième dispositifs déflecteurs (6L, 6R; 42L, 45L, 42R, 45R) présentent respectivement deux surfaces de miroir optique formant ensemble respectivement un angle (δ1, δ2) d'entre 85° et 95°, de préférence de 90°.

9. Système optique stéréoscopique (1, 1'; 1") selon l'une quelconque des revendications 1 à 8, dans lequel les premier et deuxième dispositifs déflecteurs (6L, 6R; 41 L, 42L, 41 R, 42R; 45L, 42L, 45R, 42R) et/ou une première et une deuxième surface de miroir des premier et deuxième éléments déflecteurs (3L, 3R; 40L, 40R) sont disposés de manière symétrique par rapport à un plan de symétrie commun (22).

10. Système optique stéréoscopique (1, 1'; 1") selon l'une quelconque des revendications 1 à 9, dans lequel un angle (β) entre une première surface de miroir du premier élément déflecteur (3L; 40L) et une deuxième surface de miroir du deuxième élément déflecteur (3R; 40R) est d'entre 50° et 130°, et en particulier d'entre 80° et 100°, et de préférence de 90°.

11. Système optique stéréoscopique (1, 1'; 1") selon l'une quelconque des revendications 1 à 10, dans lequel le système (1; 1'; 1") présente un système d'irradiation avec une source de rayonnement (25) pour émettre un rayonnement et avec une optique d'irradiation (26) pour fournir un chemin optique d'irradiation (27) pour le rayonnement émis par la source de rayonnement (25).

12. Système optique stéréoscopique (1, 1'; 1") selon la revendication 11,
dans lequel les première et deuxième surfaces de miroir des premier et deuxième éléments déflecteurs (3L, 3R; 40L, 40R) présentent respectivement au moins localement une plus grande transparence au rayonnement dirigé par le chemin optique d'irradiation (27) à celle au rayonnement dirigé sur le chemin optique de reproduction gauche ou droit (14L, 14R) et/ou sont espacées l'une de l'autre d'une distance (A) supérieure à zéro; et
dans lequel le chemin optique d'irradiation (27) fourni par l'optique d'irradiation (26) passe à travers la transparence accrue des première et deuxième surfaces de miroir et/ou à travers la distance (A) entre les deux première et deuxième surfaces de miroir.

13. Système optique stéréoscopique (1'; 1") selon l'une quelconque des revendications 1 à 12, dans lequel au moins un élément déflecteur (3L, 3R, 10L, 10R; 40L, 40R, 10L, 10R) et/ou au moins un dispositif déflecteur (6L, 6R; 41 L, 41 R, 42L, 42R; 45L, 45R, 42L, 42R) des premier et deuxième sous-systèmes optiques (2L, 2R) présente respectivement une surface de miroir courbe (44L, 44R, 46L, 46R) d'un rayon de courbure (m1L, m2L, m1 R, m2R) inférieur à dix mètres et de préférence inférieur à un mètre, et ladite surface de miroir courbe (44L, 44R, 46L, 46R) présente un effet de symétrie de révolution par rapport au chemin optique de reproduction (14L, 14R) respectivement convolué par celle-ci.

14. Système optique stéréoscopique (1'; 1") selon l'une quelconque des revendications 1 à 13,
dans lequel le premier sous-système optique (2L) présente en outre un premier système oculaire (11 L, 12L) et le deuxième sous-système optique (2R) présente en outre un deuxième système oculaire (11 R, 12R); et
dans lequel un rayon principal du chemin optique de reproduction gauche (14L) dirigé dans le premier système oculaire (11 L, 12L), ainsi qu'un rayon principal du chemin optique de reproduction droit (14R) dirigé dans le deuxième système oculaire s'étendent substantiellement en parallèle l'un à l'autre.

15. Système optique stéréoscopique (1, 1'; 1") selon l'une quelconque des revendications 1 à 14, dans lequel les rayons principaux des chemins optiques de reproduction gauche et droit (14L, 14R) se trouvent substantiellement dans un plan commun.
